# EUROPEAN PATENT APPLICATION

(11) **EP 4 371 569 A1**
(43) Date of publication of application: **22.05.2024**
(21) Application number: 22383104.1
(22) Date of filing: 16.11.2022
(51) Int. Cl.: A61K 39/00, A61K 39/385, C07K 14/005, C07K 14/47, C07K 14/82, C12N 7/04, C12N 15/866

(54) **VLPS AGAINST ACUTE MYELOID LEUKAEMIA**

(71) Applicant: Universidad del País Vasco/Euskal Herriko Unibertsitatea, 48940 Leoia, Bikaia (ES)
(72) Inventor: BAÑUELOS RODRÍGUEZ, Sonia, E-48940 Leioa, Vizcaya (ES); GUÉRIN AGUILAR, Diego Marcelo, E-48940 Leioa, Vizcaya (ES)
(74) Representative: ABG Intellectual Property Law, S.L.

(57) **Abstract**

The present invention belongs to the field of human therapies as it proposes the use of a viral capsid as antigen carrier for making vaccines. The invention involves the design of virus-like particles (VLPs) comprising an acute myeloid leukaemia (AML)-specific antigen and the process for their production.

## Description

### FIELD OF THE INVENTION

The present invention belongs to the field of human therapies as it proposes the use of a viral capsid as antigen carrier for making vaccines. The invention involves the design of virus-like particles (VLPs) comprising an acute myeloid leukaemia (AML)-specific antigen and the process for their production.

### BACKGROUND OF THE INVENTION

Acute myeloid leukaemia (AML), characterized by the rapid proliferation of immature myeloid cells in the bone marrow with impaired differentiation, is the most common form of leukaemia in adults. Standard treatments for AML currently include chemotherapy and allogenic stem cell transplantation. However there are frequent relapses with persistence of AML stem cells, causing a poor 5-years survival rate (ca. 27%), which makes imperative to find alternative therapies. Immunotherapy, based on harnessing the power of T cells to fight against the tumour, is emerging for the treatment of many cancers. The immunotherapeutic strategies currently under development in the case of AML include the use of monoclonal antibodies, adoptive chimeric antigen receptor -T (CART) cells and alloreactive NK (natural killer) cells, checkpoint blockade, and vaccination. On the other hand, recent progress in the understanding of the molecular basis of the disease, e.g. the AML-associated mutations, paves the way for the development of molecular targeted therapies.

Mutations of the protein nucleophosmin (NPM1) are the most common genetic lesion linked to AML, accounting for approximately one third of all patients, and reaching 50-60% of the cases with normal karyotype. The ca. 50 different mutations consist of a frameshift at the end of the NPM1 gene that alters the C-terminal sequence of the protein. This alteration causes the defective folding and trafficking of the protein, resulting in its aberrant localization in the cytoplasm instead of the nucleolus. Such feature is characteristic of this subtype of AML, thus denoted as "AML - NPM c+", which has been defined as a distinct leukaemia entity according to the World Health Organization (WHO). These genetic lesions are "driver mutations" for AML, and the ectopic sequences acquired by the protein are considered cancer "neoantigens", i.e. novel tumour-specific epitopes than can elicit an immunogenic response. Therefore, they represent valuable targets for immunotherapy of AML.

AML-associated antigens have been either loaded into dendritic cells or presented in peptide form for their use in therapeutic vaccination. However, their presentation in nanoparticle carriers such as virus-like particles (VLPs) may be favourable for several reasons. Moreover, other antigens different from NPM1 have been exploited so far, while NPM1 epitopes, never used thus far are, in principle, advantageous since they are (a) clonal, driver mutations probably present in all tumour cells, and (b) differ from any other human protein sequence.

However, despite the efforts made to date, there still exists a continuing need in the art for alternative therapeutic approaches for the treatment of AML.

### SUMMARY OF THE INVENTION

The inventors have developed a system for AML-specific antigen presentation based on VLPs derived from the insect viral pathogen *Triatoma virus* (TrV) (*Dicistroviridae: Triatovirus, Dicistroviridae* family, *Triatovirus* genus). This antigen is provided as a fusion protein with the viral structural proteins of the TrV or is connected to these proteins by a linker region. This antigen presenting system allows epitope exposure on the internal or external surface of the VLPs by the insertion or substitution of amino acids or by the chemical conjugation of the antigen, thus facilitating their recognition by the immune system and/or increasing their immunogenicity.

In a first aspect, the present invention relates to a virus-like particle (VLP) comprising
(i) the viral structural proteins VP1, VP2, VP3 and VP4, or the viral structural proteins VP1, VP2, and VP0, or the structural protein precursor (P1) of *Triatoma virus* (TrV), or a functionally equivalent variant of any of the above, and
(ii) an acute myeloid leukaemia (AML) specific antigen
wherein said antigen is provided as a fusion protein with at least one of said viral structural proteins or wherein said antigen is connected to at least one of the said viral proteins by a linker region.

In a second aspect, the present invention relates to a polynucleotide encoding a fusion protein selected from the group consisting of
(i) a fusion protein comprising a structural protein from a virus of the *Dicistroviridae* family selected from the group consisting of VP1, VP2, VP3, VP4 and VP0, or a functionally equivalent variant thereof, and an AML-specific antigen, or
(ii) a fusion protein comprising a structural protein precursor (P1) from a virus of the *Dicistroviridae* family, or a functionally equivalent variant thereof, and an AML-specific antigen.

In a third aspect, the present invention relates to a polynucleotide encoding a fusion protein selected from the group consisting of:
(i) a fusion protein comprising a structural protein from a virus of the *Dicistroviridae* family selected from the group consisting of VP1, VP2, VP3, VP4 and VP0, or a functionally equivalent variant thereof, and a first member of a binding pair, or
(ii) a structural protein precursor (P1) from a virus of the family, or a functionally equivalent variant thereof, and a *Dicistroviridae* first member of a binding pair.

In a further aspect, the invention relates to a vector comprising the polynucleotide encoding a fusion protein as above.

In a further aspect, the invention relates to a host cell comprising the polynucleotide encoding a fusion protein or the vector as above.

In a further aspect, the invention relates to a process for obtaining the *Triatoma virus* (TrV) VLP modified to express an AML-specific antigen comprising the steps:
(i) contacting a host cell with a first polynucleotide which encodes a fusion protein comprising an AML-specific antigen and the structural protein precursor (P1) of TrV or a functionally equivalent variant thereof, wherein optionally the host cell is additionally contacting with a polynucleotide comprising a sequence encoding the non-structural protein precursors (NS) of TrV or a functionally equivalent variant thereof, under conditions suitable for the entry of said polynucleotide into the cell;
(ii) maintaining the cell under conditions suitable for the expression of the polynucleotide introduced in the cell in step (i), and
(iii) recovering the VLPs from the culture obtained.

In a further aspect, the invention relates to a process for obtaining a *Triatoma virus* (TrV) VLP modified to contain an AML-specific antigen comprising the steps:
(i) contacting a host cell with a first polynucleotide which encodes a fusion protein comprising a second member of a binding pair and the structural protein precursor (P1) of TrV or a functionally equivalent variant thereof, wherein optionally the host cell is additionally contacting with a polynucleotide comprising a sequence encoding the non-structural protein precursors (NS) of TrV or a functionally equivalent variant thereof, under conditions suitable for the entry of said polynucleotide into the cell;
(ii) maintaining the cell under conditions suitable for the expression of the polynucleotide introduced in the cell in step (i), and
(iii) recovering the VLPs from the culture obtained
(iv) contacting the VLPs recovered in step (iii) with the AML-specific antigen wherein the AML-specific antigen is provided as a fusion protein with a first member of a binding pair under conditions adequate for the interaction between said first and second members of the binding pair to occur.

In a further aspect, the invention relates to a process for obtaining a *Triatoma virus* (TrV) VLP modified to contain an AML-specific antigen comprising the steps:
(i) contacting a host cell with a first polynucleotide which encodes the structural protein precursor (P1) of TrV or a functionally equivalent variant thereof, wherein optionally the host cell is additionally contacting with a polynucleotide comprising a sequence encoding the non-structural protein precursors (NS) of TrV or a functionally equivalent variant thereof, under conditions suitable for the entry of said polynucleotide into the cell;
(ii) maintaining the cell under conditions suitable for the expression of the polynucleotide introduced in the cell in step (i), and
(iii) recovering the VLPs from the culture obtained
(iv) treating the VLPs obtained in step (iii) with a reagent that creates functional groups on the VLP thereby creating functionalized VLPs and reacting the functionalized VLPs with a funcionallized form of the AML-specific antigen which functional groups which are reactive with the functional groups present in the funcionalized VLPs or, alternatively, contacting the VLPs obtained in step (iii) with the AML-specific antigen in the presence of a bifunctional reagent that is capable of reacting with groups present in the VLP and in the AML-specific antigen thereby cross-linking the VLP and the antigen.

In a further aspect, the invention relates to the VLP obtainable by the process above.

In a further aspect, the invention relates to a vaccine or immunogenic composition comprising the VLP, the polynucleotide or the vector as above.

In a further aspect, the invention relates to a VLP, to a polynucleotide, or to a vector as mentioned above for use in the treatment of an AML.

In a further aspect, the invention relates to a VLP, to a polynucleotide or to a vector as mentioned above for use in the treatment of the AML subtype characterized by presence of recurrent mutation of nucleophosmin-1 (NPM1).

### BRIEF DESCRIPTION OF THE DRAWINGS

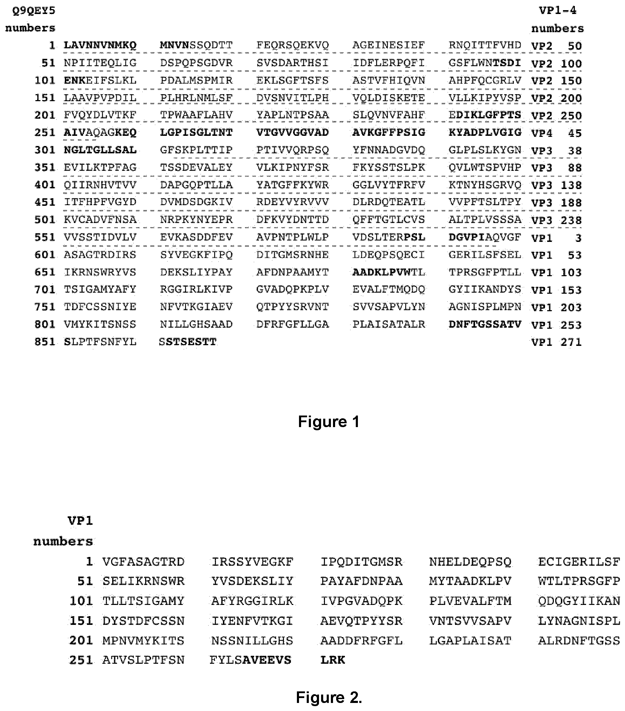

**Figure 1****.** Amino acid sequence (in one letter code) of TrV structural proteins. Left column indicates the numbers according to UniProtKB sequence code Q9QEY5, which corresponds to numbers of P1 precursor protein of VP1-VP4 structural proteins. Residue numbers referred to each of the VP1-4 proteins are indicated in the right column (regions corresponding to VP2 and VP3 are underlined). Stretches of amino acids in bold letter are the regions of insertions/substitutions.
**Figure 2****.** Amino acid sequence of the example vaccine against AML-NPMc+. Amino acids in one letter code corresponding to protein VP1 modified at the C-terminal to insert the NPM1 epitope AVEEVSLRK. For making this prototype, WT VP1 amino acids STSESTT (from Ser862 to Thr868 in sequence code Q9QEY5; see Figure 1) were replaced.

### DETAILED DESCRIPTION OF THE INVENTION

The authors of the present invention have developed VLPs based on the *Triatoma virus* (TrV), an insect virus of the *Dicistroviridae* family, wherein said VLPs comprise a AML-specific antigen of interest.

In particular, the inventors have used a recombinant virus like particle (VLP) to present an AML-linked specific antigen corresponding to the C-terminal part of the oncoprotein nucleophosmin (NPM1). Such chimeric VLPs decorated with NPM1 motifs are advantageous because a) they display a relevant epitope, b) they display it in a repetitive pattern, expected to elicit both humoral and cellular responses; c) they have immunogenic and adjuvant capabilities per se; d) they are safe for humans because they lack the viral genome and they cannot replicate nor cause an infection.

### Definitions

The term acute myeloid leukemia or AML refers to a type of cancer of the myeloid line of blood cells, characterized by the rapid growth of abnormal cells that build up in the bone marrow and blood and interfere with normal blood cell production. Risk factors include smoking, previous chemotherapy or radiation therapy, myelodysplastic syndrome, and exposure to the chemical benzene. The underlying mechanism involves replacement of normal bone marrow with leukemia cells, which results in a drop in red blood cells, platelets, and normal white blood cells. Diagnosis is generally based on bone marrow aspiration and specific blood tests. AML has several subtypes for which treatments and outcomes may vary. The first-line treatment of AML is usually chemotherapy, with the aim of inducing remission. People may then go on to receive additional chemotherapy, radiation therapy, or a stem cell transplant. The specific genetic mutations present within the cancer cells may guide therapy, as well as determine how long that person is likely to survive. The French-American-British (FAB) classification system divides AML into eight subtypes, M0 through to M7, based on the type of cell from which the leukemia developed and its degree of maturity. Classification is done by examining the appearance of the malignant cells with light microscopy and/or by using cytogenetics to characterize any underlying chromosomal abnormalities.

The term "5'-end" designates the end of a nucleotide strand that has the fifth carbon in the sugar-ring of the deoxyribose at its terminus.

The term "3'-end" designates the end of a nucleotide strand that has the hydroxyl group of the third carbon in the sugar-ring of the deoxyribose at its terminus.

The term "biotin acceptor peptide" or "BAP", as used herein, relates to a peptide with the capacity to bind to biotin or to an analog thereof (e.g. 2-iminobiotin, etc.). Illustrative, non-limiting, examples of BAP are mentioned in EP711303. Many biotinylated ligands are commercially available or can be prepared by standard methods. Processes for coupling a biomolecule, e. g. a protein molecule, to biotin are well known in the art (Bayer and Wilchek 1992 Methods in Mol Biol 10, 143).

The term "biotin-binding molecule", as used herein, relates to a member of a binding pair that binds to biotin. Particularly, the biotin-binding molecule is avidin, streptavidin (SA) or avidin fragments which retain substantial binding activity for biotin, such as at least 50 percent or more of the binding affinity of native SA. As used herein, the term "avidin" refers to a glycoprotein found in egg whites and in tissues of birds, reptiles and amphibian's protein and which has the capacity to bind to biotin with high affinity as well as any expressed or engineered form of the avidin biotin-binding molecule, such as streptavidin, neutravidin and the like. The term avidin includes both avidin found naturally in the eggs of *Gallus gallus* (NCBI accession numbers NM_205320.1 / GL45384353en, release as of 14 May 2013) as well as the orthologues of said protein in other species. Streptavidin, corresponding to the protein from *Streptomyces avidinii* (accession number CAA00084.1 in GenBank, release as of 28 January 1993), as well as the orthologues, homologues and fragments of streptavidin defined in the same manner as avidin. Streptavidin comprises 4 subunits each of which contains a binding site for biotin. Streptavidin (SA) or avidin fragments which retain substantial binding activity for biotin, such as at least 50 percent or more of the binding affinity of native SA or avidin, respectively, may also be used. Preferably, the affinity of the avidin variant for biotin is of at least 10¹⁵ M⁻¹, 10¹⁴ M⁻¹, 10¹³ M⁻¹, 10¹² M⁻¹, 10¹⁰ M⁻¹ or 10⁹ M⁻¹.

Avidin and streptavidin variants suitable for use in the present invention include, without limitation:
- "Core streptavidin" ("CSA"), which is a truncated version of the full-length streptavidin polypeptide which may include streptavidin residues 13-138, 14-138, 13-139 and 14-139. See, e.g., Pahler et al., (J. Biol. Chem. 1987, 262: 13933-37).
- Truncated forms of streptavidin and avidin that retain strong binding to biotin (See, e.g. Sano et al., (J Biol Chem., 1995, 270: 28204-09) (describing core streptavidin variants 16-133 and 14-138) (U.S. Pat. No. 6,022,951).
- Mutants of streptavidin and core forms of streptavidin which retain substantial biotin binding activity or increased biotin binding activity. See Chilcoti et al., Proc Natl. Acad. Sci. USA. Feb. 28, 1995; 92(5):1754-8; Reznik et al., Nat Biotechnol. August 1996; 14(8):1007-1 1.
- Mutants with reduced immunogenicity, such as mutants mutated by site-directed mutagenesis to remove potential T cell epitopes or lymphocyte epitopes. See Meyer et al., Protein Sci. 2001 10: 491-503.
- Mutants of avidin and core forms of avidin which retain substantial biotin binding activity or increased biotin binding activity also may be used. See Hiller et al., J. Biochem. (1991) 278: 573-85; Livnah et al. Proc. Natl. Acad. Sci. USA (90: 5076-80 (1993).
- Variants resulting from the chemical modification of avidin such as those resulting from the complete or partial modification of glycosylation and fragments thereof as well as the completely deglycosylated avidin variant known as neutravidin.
- Avidin mutants as described in WO05047317A1
- Avidin-like proteins as described in WO06045891,
- Recombinant avidin as described in WO0198349,
- Avidin variants as described in WO0027814,
- Monomeric streptavidin as described in WO06084388,
- Modified streptavidin dimers such as those described in WO06058226,
- The protein with biotin binding capacity as described in WO04018509,
- Streptavidin having a higher affinity for biotin as described in WO9840396,
- The modified streptavidin and avidin molecules as described in WO9640761,
- The streptavidin mutants as described in WO9711183,
- The streptavidin with modified affinity as described in WO9624606.

For convenience, in the instant description, the terms "avidin" and "streptavidin" as used herein are intended to encompass biotin-binding fragments, mutants and core forms of these binding pair members. Different avidin variants are commercially available, such as Extravidin (Sigma-Aldrich), NeutrAvidin (Thermo Scientific), NeutrAvidin (Invitrogen) and NeutraLite (Belovo). Moreover, the nucleic acid sequences encoding streptavidin and avidin and the streptavidin and avidin amino acid sequences can be found, for example, in GenBank Accession Nos. X65082; X03591; NM --205320; X05343; Z21611; and Z21554.

The expression "capable of interacting specifically" or "specific interaction", as used herein in the context of the first and second members of a binding pair, describes the interaction between a first species and a second species characterized in that the nature of the binding is such that an antibody, a receptor or a nucleic acid binding protein binds to its corresponding binding partner but does not substantially bind to other species. Likewise, as used herein, the term "binding" or "binding to" means the physical association between a first species and a second species, for example, the binding of a ligand by a receptor, the binding of an antigen by an antibody, the binding of a nucleic acid by a nucleic acid binding protein, etc.

The term *"Dicistroviridae* family", as used herein, relates to a family of Group IV (positive sense single stranded RNA) arthropod-infecting viruses, particularly insect-infecting viruses. Some insects commonly infected by dicistroviruses include, without limitation, aphids, leafhoppers, flies, bees, ants, crickets, triatomines and silkworms. Viruses of this family are characterized by the location of their structural protein genes at the 3' end of the genome, and by having two genomic segments. The *Dicistroviridae* family includes *Aparavirus* and *Cripavirus* genuses. In a particular embodiment, the insect from the *Dicistroviridae* family is an insect from the *Triatovirus* genus, more particularly a *Triatoma virus* (TrV).

The term *"Triatoma* virus", also referred to as TrV, is a viral pathogen of the blood-sucking reduviid bug *Triatoma infestans* (*Hemiptera: Reduviidae,* family *Hemiptera*, genus *Reduviidae*), the main transmitter of human Chagas disease (also called American trypanosomiasis), as well as other species of triatomine insects (popularly named kissing bugs). This disease is a major health problem in Latin America, where it is endemic and affects about eight million people, 30-40% of whom develop cardiomyopathy and/or digestive megasyndromes. The aetiological agent of Chagas disease is the protozoan parasite *Trypanosoma cruzi*, which infects the insect vector, which in turn infects vertebrate hosts during blood meals. TrV is the type species of *Triatovirus* genus in the *Dicistroviridae* family, which is a family of small ssRNA(+) viruses of invertebrates. The members of the *Dicistroviridae* family are non-enveloped positive-sense single-stranded RNA (+ssRNA) viruses pathogenic to beneficial arthropods as well as insect pests of medical importance. The *Triatoma* virus belongs to the group of RNA viruses requiring a RNA polymerase for its replication, i.e. is a virus the genome of which is formed by an RNA molecule and which, unlike other RNA viruses the genome of which first undergoes a reverse transcription to give rise to a DNA which is used as a template for the replication, is replicated as a result of a RNA-dependent RNA polymerase which is encoded by the genome of the virus itself.

The term "epitope" as used herein, refers to that portion of a given immunogenic substance that is the target of, i.e., is bound by, an antibody or cell-surface receptor of a host immune system that has mounted an immune response to the given immunogenic substance as determined by any method known in the art. Further, an epitope may be defined as a portion of an immunogenic substance that elicits a humoral or cellular response, in particular an antibody response or a T-cell response in an animal, as determined by any method available in the art (see, for example, Geysen et al., Proc Natl Acad Sci (1984) Vol.81 3998-4002). An epitope can be a portion of any immunogenic substance, such as a protein, polynucleotide, polysaccharide, an organic or inorganic chemical, or any combination thereof.

The term "fusion protein" as used herein, relates to proteins generated by gene technology which consist of two or more functional domains derived from different proteins. A fusion protein may be obtained by conventional means (e.g. by means of gene expression of the nucleotide sequence encoding for said fusion protein in a suitable cell). The fusion protein of the invention comprises an AML-specific antigen of interest and at least one viral structural protein, in particular at least one viral structural protein of the *Triatoma* virus selected from the group comprising VP0, VP1, VP2, VP3 and VP4.

The term "AML-specific antigen" as used herein, relates to peptides or proteins associated with Acute myeloid leukaemia (AML), which are capable of (suitable or designed for) inducing an immune response against AML, therefore the AML-specific antigen can be used in the treatment of AML by means of the stimulation of an antigen-specific immune response against an AML antigen.

The term "host cell", as used herein, refers to a cell into which a nucleic acid of the invention, such as a polynucleotide or a vector according to the invention, has been introduced. The terms "host cell" and "recombinant host cell" are used interchangeably herein. It should be understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

The term "immunogenic composition", as used herein, refers to a composition that elicits an immune response in a subject that produces antibodies or cell-mediated immune responses against a specific immunogen. The term "antigenic composition" refers to a composition that can be recognized by a host immune system. For example, an antigenic composition contains epitopes that can be recognized by humoral or cellular components of a host immune system.

The term "ligand" according to the present invention refers to a molecule that is capable of interacting with and binding to the AML-specific antigen which is modified so as to contain a ligand-binding region, thereby resulting in the decoration of the VLP with one or more copies of the AML-specific antigen. Said ligand contains a region which is capable of specifically interact with a specific ligand-binding domain that is present on the AML-specific antigen, thereby resulting in the binding of the AML-specifici antigen to the VLP by virtue of the interaction between the ligand present in the VLP and the ligand-binding domain present in the AML-specific antigen.. By way of illustration, the "ligand" and "ligand binding" pair acts as a specific binding pair, e.g., of the type of biotin-avidin, epitope-antibody or functional fragment thereof, in which each of said peptide insert and ligand form the members of said specific binding pair, each of them containing the binding domains or the corresponding regions of interaction; thus, depending on the ligand binding region present in the AML-specific antigen, the ligand which must be present in VLP will be chosen, and, vice versa, depending on the ligand which is to be bound to the VLP of the invention, a specific ligand-binding region would have to be included in the AML-specific antigen so as to allow the binding of the VLP to the AML-specific antigen.

In a particular embodiment, the binding of the ligand to the AML-specific antigen is performed directly. In this case, for its use in the present invention, a product of interest with the capacity to bind to said antigen present in the VLP of the invention will be used, such that said AML-specific antigen and product of interest form a specific binding pair. By way of non-limiting illustration, when said AML-specific antigen comprises an amino acid tag (e.g., a histidine tag (his-tag), an arginine tag (arg-tag), etc.); likewise when said peptide insert comprises a peptide epitope which can be recognized by an antibody (e.g., c-myc-tag, etc.), the product of interest is an antibody or a functional fragment thereof; etc.

Alternatively, the binding of said ligand to the AML-specific antigen can be performed indirectly, i.e., by means of using a "linker", or molecule comprising, on one hand, a first region of interaction with the binding domain contained in the AML-specific antigen present in the VLP and, on the other hand, a second region of interaction (binding domain) with a region of interaction present in the ligand. Thus, the product of interest binds to the VLP through a structure of the type: AML-specific antigen -linker-ligand-product of interest. Illustrative non-limiting examples of this embodiment include the use of avidin or an analog thereof (linker) when the AML-specific antigen present in the VLP comprises a BAP; etc. As used herein, the term "avidin" includes any avidin, of a eukaryotic or prokaryotic origin, and its variants which maintain the capacity to bind to biotin; in a particular embodiment, the ligand present in the conjugate of the invention comprises a monomeric variant of avidin (mAV).

Alternatively, the indirect binding of said ligand to the AML-specific antigen can be performed by means of the reaction between two different functional groups found within the VLP and the AML-associated antigen or can be the result of the cross-linking of the AML-associated antigen and the VLP by a bifunctional cross-linking.

The term "functional group", as used in the context of the present invention refers to a group of atoms in a molecule with distinctive chemical properties, regardless of the other atoms in the molecule. The atoms in a functional group are linked to each other and to the rest of the molecule by covalent bonds. Functional groups can also be charged, e.g. in carboxylate salts (-COO-), which turns the molecule into a polyatomic ion or a complex ion.

The term "bifunctional crosslinker" or a "crosslinking agent", as used in the context of the present invention refers to a type of chemical reagent that has two or more functional groups at its "ends". A bifunctional crosslinker is a molecule with two or more functional groups that are used to cross-link two or more molecules together.

The term "non-structural protein", as used in the context of the present invention in relation to a virus of the *Dicistroviridae* family, in particular to a *Triatoma* virus, relates to proteins encoded by the viral genome which do not form the capsid of said virus. The dicistrovirus genome has two open reading frames, namely ORF1 and ORF2, which encodes the non-structural protein precursor (NS) and the structural protein precursor (P1), respectively. *Dicistrovirus*, in particular TrV, non-structural proteins include the viral protease, the viral helicase, a small genome-linked protein (VPg), and RNA-dependent RNA polymerase (RdRp).

The term "non-structural protein precursor" or "NS", as used in the present invention in relation to a virus of the *Dicistroviridae* family, in particular to a *Triatoma* virus, relates to the non-structural polyprotein precursor encoded by the 5' ORF1 viral genome and, particularly, corresponding to the region 549-5936 of *Triatoma* virus complete RNA genome according to the sequence NC_003783.1 (NCBI database as of April 28th, 2010), to the region 549-5936 of the sequence AF178440 from the non-structural and capsid protein precursors (NCBI database as of April 14th, 2010), and according to Czibener C et al. 2000 J Gen Virology 81: 1149-1154. The amino acid sequence of the TrV non-structural polyprotein precursor is located in NCBI database under accession number NP_620562.1 (1795 aa, NCBI database as of April 28th, 2010). From this non-structural protein precursor, a RNA helicase, a peptidase and a RNA-dependent RNA polymerase (which catalyzes the synthesis of the RNA strand complementary to a given RNA template) are produced.

The term "polynucleotide", as used in the present invention, relates to a polymer formed by a variable number of monomers wherein the monomers are nucleotides, including ribonucleotides as well as deoxyribonucleotides. The polynucleotides include monomers modified by methylation as well as unmodified forms. The terms "polynucleotide" and "nucleic acid" are used indiscriminately in the present invention and include mRNA, cDNA and recombinant polynucleotides. As used in the present invention, polynucleotides are not limited to polynucleotides as they appear in nature, and also include polynucleotides where unnatural nucleotide analogues and inter-nucleotide bonds appear. Non-limitative examples of this type of unnatural structures include polynucleotides wherein the sugar is different from ribose, polynucleotides wherein the phosphodiester bonds 3'-5' and 2'-5' appear, polynucleotides wherein inverted bonds (3'-3' and 5'-5') appear and branched structures. Also, the polynucleotides of the invention include unnatural inter-nucleotide bonds such as peptide nucleic acids (PNA), locked nucleic acids (LNA), C1-C4 alkylphosphonate bonds of the methylphosphonate, phosphoramidate, C1-C6 alkylphosphotriester, phosphorothioate and phosphorodithioate type.

The term "structural protein", as used in the present invention in the context of the *Triatoma* virus proteins, relates to those proteins forming the viral capsid of TrV. The dicistrovirus genome has two open reading frames, namely ORF1 and ORF2, which encodes the non-structural proteins (NS) and the structural protein precursor (P1), respectively. TrV structural proteins include VP1, VP2, VP3 and VP4. VP0 is the precursor of VP3 and VP4.

The term "structural protein precursor" or P1, as used in the present invention in relation to *Triatoma* virus, relates to a protein precursor encoded by the region 6109-8715 of Triatoma virus RNA genome according to the sequence NC_003783.1 (NCBI database as of April 28th, 2010), by the region 6109-8715 of the sequence AF178440 from the non-structural and capsid protein precursors (NCBI database as of April 14th, 2010), and according to Czibener C et al. 2000 J Gen Virology 81: 1149-1154. The amino acid sequence of the structural protein precursor is located at NCBI database under accession number NP_620563.1 (868 aa, NCBI database as of April 28th, 2010). In one embodiment, the structural protein precursor contains a modified version of the sequence of the database NP_620563.1 containing the aminoacid substitutions H49D in VP2 and V54M, V128I, K202E in VP3. In another embodiment, the structural protein precursor may naturally contain other amino acid substitutions such as V190I and L191F in VP1, A75T in VP2, and V163F and E206G in VP3. The structural protein precursor is processed during viral biogenesis to yield the different polypeptides that form the viral capsid, namely, VP1, VP2, VP3, VP4 and VP0 (see Agirre et al., 2011, Virology, 409: 91-101).

The term "vaccine", as used herein, relates to an immunogenic composition for *in vivo* administration to a host, especially a human host, to confer protection against a disease.

The term "vector", as used herein, refers to a nucleic acid sequence comprising the necessary sequences so that after transcribing and translating said sequences in a cell a viral particle is generated. Said sequence is operably linked to additional segments that provide for its autonomous replication in a host cell of interest. Preferably, the vector is an expression vector, which is defined as a vector, which in addition to the regions of the autonomous replication in a host cell, contains regions operably linked to the genome of the invention and which are capable of enhancing the expression of the products of the genome according to the invention. The vectors of the invention can be obtained by means of techniques widely known in the art. See Brown T, "Gene Cloning" (Chapman & Hall, London, GB, 1995); Watson R, et al., "Recombinant DNA", 2nd Ed. (Scientific American Books, New York, NY, US, 1992); Alberts B, et al., "Molecular Biology of the Cell" (Garland Publishing Inc., New York, NY, US, 2008); Innis M, et al., Eds., "PCR Protocols. A Guide to Methods and Applications" (Academic Press Inc., San Diego, CA, US, 1990); Erlich H, Ed., "PCR Technology. Principles and Applications for DNA Amplification" (Stockton Press, New York, NY, US, 1989); Sambrook J, et al., "Molecular Cloning. A Laboratory Manual" (Cold Spring Harbor Laboratory Press, Cold Spring Harbor, NY, US, 1989); Bishop T, et al., "Nucleic Acid and Protein Sequence. A Practical Approach" (IRL Press, Oxford, GB, 1987); Reznikoff W, Ed., "Maximizing Gene Expression" (Butterworths Publishers, Stoneham, MA, US, 1987); Davis L, et al., "Basic Methods in Molecular Biology" (Elsevier Science Publishing Co., New York, NY, US, 1986), Schleef M, Ed., "Plasmid for Therapy and Vaccination" (Wiley-VCH Verlag GmbH, Weinheim, DE, 2001).

The term "viral particle", or "virion" as used herein, refers to a whole viral particle and not to a protein subunit or peptide. Viral particles consist of two or three parts: the genetic material of the virus made from either DNA or RNA; a protein coat that protects these genes; and, in some cases, an envelope of lipids that surrounds the protein coat when they are outside a cell. The shape of the viral particle ranges from simple helical and icosahedral forms to more complex structures, depending on the virus.

The term "virus-like particle", also referred to as "VLP", relates to non-infectious particles resembling viruses that do not contain any viral genetic material. VLPs are the result of the expression of viral structural proteins, such as capsid proteins, and their self-assembly.

### Virus-like particles

The authors of the present invention have developed a system for AML-specific antigen presentation based on VLP derived from the *Triatoma* virus (TrV).

Thus, in a first aspect, the present invention relates to a virus-like particle (VLP) (hereinafter the VLPs of the invention) comprising:
(i) the viral structural proteins VP1, VP2, VP3 and VP4, or the viral structural proteins VP1, VP2, and VP0, or the structural protein precursor (P1) of *Triatoma virus* (TrV), or a functionally equivalent variant of any of the above, and
(ii) an acute myeloid leukaemia (AML) specific antigen
wherein said antigen is provided as a fusion protein with at least one of said viral structural proteins or wherein said antigen is connected to at least one of the said viral proteins by a linker region.

A VLP according to the invention is an oligomeric structure resulting from the assembly of a determined number of TrV capsid structural proteins. In a preferred embodiment, the VLPs are formed by the assembly of VP1, VP2, VP3 and VP4. In a still more preferred embodiment, the VLP is a structure of icosahedral symmetry, with a pseudo-triangulation number *P* = *3*, formed by 60 protomers made up of proteins VP1 (corresponding to residues 598-868 in Q9QEY5 sequence, 271 aa; see Figure1), VP2 (corresponding to residues 1-255 in Q9QEY5 sequence, 255 aa) and VP3 (corresponding to residues 313-597 in Q9QEY5 sequence, 285 aa), wherein VP1 is located around the fivefold axes, and VP2 and VP3 alternate around the threefold icosahedral axes. In a particular embodiment, the VLP according to the invention comprises the structural proteins VP1, VP2, VP3 and VP4 of a *Triatoma* virus (TrV), wherein said structural proteins VP1, VP2 and VP3 are in a stoichiometry of 1:1:1. In a preferred embodiment, the VLP according to the invention comprises 60 units of VP1, 60 units of VP2 and 60 units of VP3.

In another preferred embodiment, the VLP is formed by an assembly of the VP1, VP2, VP3 and VP4 polypeptides.

In another preferred embodiment, the VP3 and VP4 polypeptides appear as an unprocessed polyprotein known as VP0. Thus, in this case, the VLPs result from the assembly of the VP1, VP2 and VP0 polypeptides. In one embodiment, the average size of the VLPs according to the invention is about 30 nm.

In another preferred embodiment, the VLP is formed by the structural protein precursor P1 from a *Triatoma* virus (TrV). In this particular embodiment, the average size of the VLPs according to the invention is about 50 nm.

The molecular description of the capsid of TrV is located at Protein Data Bank under accession number 3NAP (Protein Data Bank as released on May 30th, 2013; DOI:10.2210/pdb3nap/pdb). The amino acid sequence of TrV P1 capsid polyprotein precursor comprises 868 residues and is located at UniProt database under accession number Q9QEY5 (UniProt database version 40, as of April 3, 2013; see Figure1). In one embodiment, the structural protein precursor contains a modified version of the sequence differs from the sequence provided in the UniProt database under accession number Q9QEY5 in that it contains the aminoacid substitutions H49D in VP2 and V54M, V128I, K202E in VP3. In another embodiment, the structural protein precursor may naturally contain other amino acid substitutions such as V190I and L191F in VP1, A75T in VP2, and V163F and E206G in VP3. The sequence of the polynucleotide encoding the nonstructural protein precursor and capsid protein precursor of *Triatoma* virus is located in NCBI database under accession No. AF178440 (as of April 14th, 2000, 9010 bp). The complete genome of the *Triatoma virus* is located at NCBI database under accession number NC_003783.1 (as of April 28th, 2010, 9010 bp).

It will be understood that the capsid protein variants forming the VLPs of the present invention are designed so that they are still capable of forming VLPs, i.e. that their assembly properties are not altered by the presence of the one or more copies of the AML-specific antigen. Methods available in the art for determining whether a capsid protein variant is capable of forming VLPs are well-known in the art. For instance, a polynucleotide encoding a modified P1 structural precursor protein comprising one or more modified capsid protein can be transfected into a cell together with the NS precursor protein. If the modified capsid protein is capable of forming VLPs, the cell will produce VLPs that can be characterized using any technique known in the art, including the techniques used for the characterization of TrV and described by Agirre et al. (supra.), native mass spectrometry (NMS) and atomic force microscopy (AFM) as described by Snidjer et al. (Snidjer J et al. 2013 Nat Chem 5(6): 502-509), techniques such as SDS-PAGE, dynamic light scattering, mass spectrometry, transmission electron microscopy as well as other techniques known in the art for detecting multiprotein assemblies such as co-immunoprecipitation, density gradient centrifugation, non-denaturing gel electrophoresis and the like. Preferably, the capsid protein variants show a viral capsid assembly activity of at least by 60%, preferably by 70%, advantageously by 80%, more preferably by 90%, more preferably by 95%, even more preferably by 97% and even more preferably by 98%, advantageously by 99% of the activity obtained with the complete protein.

The invention contemplates also VLPs wherein the TrV VP1, VP2, VP3, and VP4, capsid proteins are not the native proteins as they appear in the TrV in nature but functionally equivalent variants thereof.

In the context of the invention, "functionally equivalent variant" of a structural protein of the *Triatoma virus* is understood as any sequence having additions, substitutions, deletions or combinations thereof in its amino acid sequence and/or which has been chemically modified with respect to said sequence and which substantially maintains the function of said structural protein, particularly the capacity of assembling into a spheroidal particle of similar size to TrV viral capsid. Suitable assays for determining viral capsid assembly capacity are known by the skilled person and include electron microscopy, cryomicroscopy, etc. as disclosed for example in Böttcher B et al. 1998 EMBO J 17(23):6839-45; and Yadav SS et al. 2012 Virology 429(2): 155-162. Preferably, the functional equivalent variants of the structural proteins of the *Triatoma* virus show the aforementioned viral capsid assembly activity at least by 60%, preferably by 70%, advantageously by 80%, more preferably by 90%, more preferably by 95%, even more preferably by 97% and even more preferably by 98%, advantageously by 99%.

The "functionally equivalent variant" of the structural proteins of the *Triatoma* virus preferably have a sequence identity with these structural proteins of at least 50%, at least 60%, at least 70%, at least 80%, at least 90%, at least 91%, at least 92%, at least 93%, at least 94%, at least 95%, at least 96%, at least 97%, at least 98% or at least 99%. The degree of identity between the variants and the natural proteins is determined by using computer algorithms and methods that are widely known for the persons skilled in the art. For example, the identity between two amino acid sequences is determined by using the BLASTP algorithm (BLASTManual, Altschul, S., et al, NCBI NLM NIH Bethesda, Md. 20894, Altschul, S., et al., J. Mol. Biol. 21 5: 403-410 (1990), though other similar algorithms can also be used.

In the context of the invention, "functional equivalent variants" of the non-structural protein precursor NS of the *Triatoma* virus show the helicase, peptidase and/or RNA-dependent RNA polymerase activities at least by 60%, preferably by 70%, advantageously by 80%, more preferably by 90%, more preferably by 95%, even more preferably by 97% and even more preferably by 98%, advantageously by 99%.

The "functionally equivalent variant" of the non- structural protein precursor NS of the *Triatoma* virus preferably have a sequence identity with this protein of at least 50%, at least 60%, at least 70%, at least 80%, at least 90%, at least 91%, at least 92%, at least 93%, at least 94%, at least 95%, at least 96%, at least 97%, at least 98% or at least 99%. The degree of identity between the variants and the natural proteins is determined by using computer algorithms and methods that are widely known for the persons skilled in the art. For example, the identity between two amino acid sequences is determined by using the BLASTP algorithm (BLASTManual, Altschul, S., et al, NCBI NLM NIH Bethesda, Md. 20894, Altschul, S., et al., J. Mol. Biol. 21 5: 403-410 (1990), though other similar algorithms can also be used.

The VLPs of the present invention comprise an AML-specific antigen. In a particular embodiment, the AML-specific antigen comprises at least one epitope. In a more particular embodiment, the AML-specific antigen comprises at least one epitope from an antigen selected from the group consisting of:
(i) an antigen derived from the nucleophosmin 1 protein,
(ii) an antigen derived from the WT1 protein,
(iii) an antigen derived from the isocitrate dehydrogenase 1 protein, and
(iv) an antigen derived from a mutant FLT3 gene comprising an internal tandem duplication.

Nucleophosmin (NPM), also known as nucleolar phosphoprotein B23 or numatrin, is a protein that in humans is encoded by the NPM1 gene. The term "antigen derived from the nucleophosmin 1 protein" refers to antigens that have less than 230 amino acids, preferably between 1 and 150 amino acids, more preferably between 1 and 80 amino acids, more preferably between 1 and 50 amino acids, more preferably between 1 and 20 amino acids, more preferably between 1 and 10 amino acids (both extremes of all values included).

The Wilms tumor protein (WT-1) is a protein in vertebrates that controls the activity of other genes as a transcription factor. The term "antigen derived from WT-1 protein" refers to antigens that have less than 449 amino acids, preferably between 1 and 150 amino acids, more preferably between 1 and 80 amino acids, more preferably between 1 and 50 amino acids, more preferably between 1 and 20 amino acids, more preferably between 1 and 10 amino acids (both extremes of all values included).

The isocitrate dehydrogenase (IDH) 1 protein is an enzyme that catalyzes the oxidative decarboxylation of isocitrate, producing alpha-ketoglutarate (a-ketoglutarate) and CO₂. The term "antigen derived from IDH1 protein" refers to antigens that have less than 414 amino acids, preferably between 1 and 150 amino acids, more preferably between 1 and 100 amino acids, more preferably between 1 and 50 amino acids, more preferably between 1 and 30 amino acids, more preferably between 1 and 20 amino acids (both extremes of all values included).

The FLT3 gene provides instructions for making a protein called fms-like tyrosine kinase 3 (FLT3), which is part of a family of proteins called receptor tyrosine kinases (RTKs). The term "antigen derived from a mutant FLT3 gene" refers to antigens that have less than 900 amino acids, preferably between 1 and 200 amino acids, more preferably between 1 and 100 amino acids, more preferably between 1 and 50 amino acids, more preferably between 1 and 20 amino acids, more preferably between 1 and 10 amino acids (both extremes of all values included).

In a particular embodiment, the epitope is selected from the group consisting of the sequences AVEEVSLRK peptide (UniProt:E5RI98 and P06748) (SEQ ID NO: 1), the RMFPNAPYL peptide from Wilms tumor protein 1 (UniProt:P19544) (SEQ ID NO: 2), the GWVKPIIIGHHAYGDQYRAT peptide (IEDB (iedb.org) epitope ID 1445707) (SEQ ID NO: 3) or the YVDFREYEYY peptide (IEDB epitope ID 858644) (SEQ ID NO: 4).

In a preferred embodiment, the AML-specific antigen is an antigen derived from the nucleophosmin 1 protein and said antigen comprises the epitope with the sequence AVEEVSLRK peptide (UniProt:E5RI98 and P06748) (SEQ ID NO: 1).

The AML-specific antigen can be provided as a fusion protein with at least one of the viral structural proteins described before or it can be connected to at least one of the said viral proteins by a linker region.

In a particular embodiment, the linker region that connects the AML-specific antigen with at least one of the viral proteins is the result of the reaction between a first member of a binding pair and a second member of the binding pair.

In a particular embodiment, the AML-specific antigen comprises a first member of a binding pair, wherein said member of a binding pair has the capacity to bind with a high affinity to a second member of a binding pair. The first member of the binding pair does not involve any particular size or any other structural technical feature different from that of said peptide (which comprises a specific binding domain) being capable of binding to the second member of the binding pair. Therefore, virtually any peptide/ligand pair wherein the peptide has the capacity to bind to the ligand can be used in the present invention as first/second members of the binding pair. By way of non-limiting illustration, said peptide can be a member of a specific binding pair, for example, an amino acid tag (e.g., a histidine tag (his-tag), an arginine tag (arg-tag), etc.), a peptide epitope which can be recognized by an antibody (e.g., c-myc-tag, etc.), a biotin acceptor peptide (BAP), a linear domain of interaction with another protein or proteins (e.g. SH3 proteins, signal transduction proteins, etc.), a post-translational modification sequence (e.g. myristoylation, methylation, phosphorylation, etc.), a sequence of transport to a cell compartment, etc.

In a particular embodiment, the AML-specific antigen in the VLP of the invention comprises a first member of a binding pair, particularly a first member of a binding pair selected from the group comprising a biotin acceptor peptide (BAP), a peptide comprising the tripeptide Arg-Gly-Asp, a peptide comprising the sequence XBBXBX, wherein X represents any amino acid and B represents Arg or Lys, and a peptide comprising the sequence XBBBXXBX, wherein X represents any amino acid and B represents Arg or Lys.

In a particular embodiment, wherein AML-specific antigen comprises a first member of the binding pair, the VLP is bound to a second member of a binding pair, said second member of the binding pair being bound to the first member of the binding pair. In a particular embodiment, the second member of the binding pair is a molecule comprising a biotin-binding region. Molecules comprising a biotin-binding region according to the invention include, without limitation, avidin, an avidin analog, streptavidin, and streptavidin analog.

In a particular embodiment, the AML-specific antigen comprises the XBBXBX motif (Verrecchio A et al. 2000 J Biol Chem 275(11): 7701-7707) as a first member of the binding pair, wherein X represents any amino acid and B represent a basic amino acid selected from the group consisting of Arg (R) and Lys (K). The second member of the binding pair is avidin, an avidin analog, streptavidin, or streptavidin analog.

In a particular embodiment, the AML-specific antigen comprises the XBBBXXBX motif (Verrecchio A et al. 2000 J Biol Chem 275: 7701-7707) as a first member of the binding pair, wherein X represents any amino acid and B represent a basic amino acid selected from the group consisting of Arg (R) and Lys (K). The second member of the binding pair is avidin, an avidin analog, streptavidin, or streptavidin analog.

In another particular embodiment, the first member of the binding pair is the biotin acceptor peptide (BAP) and the second member of the binding pair is avidin, an avidin analog, streptavidin, or streptavidin analog.

Illustrative, non-limitative, examples of BAP are mentioned in EP711303 and include peptides comprising the following amino acid sequence:
LX₁X₂IX₃X₄X₅X₆KX₇X₈X₉X₁₀ wherein X₁ is any amino acid; X₂ is an amino acid different from Leu, Val, Ile, Trp, Phe, or Tyr; X₃ is Phe or Leu; X₄ is Glu or Asp; X₅ is Ala, Gly, Ser, or Thr; X₆ is Gln or Met; X₇ is Ile, Met, or Val; X₈ is Glu, Leu, Val, Tyr, or Ile; X₉ is Trp, Tyr, Val, Phe, Leu, or Ile; and X₁₀ is any amino acid different from Asp or Glu.

In a particular embodiment, the amino acid sequence of BAP is the following 15 amino acid sequence:
GLNDIFEAQKIEWHE (SEQ ID NO: 8)

BAP is recognized by biotin ligase (BL) encoded by *Escherichia coli* gene BirA. The presence of this peptide in recombinant proteins allows the specific ligation of a biotin molecule to such proteins. This approach has allowed developing systems for purifying soluble proteins and protein complexes.

In another particular embodiment, the linker region that connects the AML-specific antigen with at least one of the viral proteins is the result of the reaction between two different functional groups found within the VLP and the AML-associated antigen.

Common functional groups are known to an expert in the field and include: hydrocarbons, groups containing halogen, groups containing oxygen, groups containing nitrogen, groups containing sulfur, groups containing phosphorus, groups containing boron and groups containing metals.

Some of the most common functional groups are: hydroxyl functional group, aldehyde functional group, ketone functional group, amine functional group, amino functional group, amide functional group, ether functional group, ester functional group, carboxylic acid functional group, thiol functional group and phenyl functional group.

The hydroxyl functional group [H-OH] is the simplest of all the common organic functional groups. Also known as the alcohol group or hydroxy group, the hydroxyl group is an oxygen atom bonded to a hydrogen atom. Hydroxy groups link biological molecules together via dehydration reactions. Hydroxyls are often written as OH on structures and chemical formulas. While hydroxyl groups are not highly reactive, they do readily form hydrogen bonds and tend to make molecules that contain them soluble in water. Examples of common compounds containing hydroxyl groups are alcohols and carboxylic acids. The hydroxyl functional group consists of a hydrogen atom attached to an oxygen atom.

The aldehyde functional groups are made up of carbon and oxygen double-bonded together and hydrogen bonded to the carbon. An aldehyde may exist as either the keto or enol tautomer. The aldehyde group is polar. Aldehydes have formula R-CHO.

A ketone is a carbon atom double bonded to an oxygen atom that appears as a bridge between two other parts of a molecule. Another name for this group is the carbonyl functional group. The aldehyde is a ketone where one R is the hydrogen atom.

The amine functional groups are derivatives of ammonia (NH₃) where one or more of the hydrogen atoms are replaced by an alkyl or aryl functional group.

The amino functional group is a basic or alkaline group. It's commonly seen in amino acids, proteins, and the nitrogenous bases used to build DNA and RNA. The amino group is NH₂, but under acidic conditions, it gains a proton and becomes NH₃⁺.

The amides functional groups are a combination of a carbonyl group and an amine functional group.

An ether functional group consists of an oxygen atom forming a bridge between two different parts of a molecule. Ethers have formula ROR.

The ester functional group is another bridge group consisting of a carbonyl group connected to an ether group. Esters have formula RCO2R.

The carboxylic acid functional group is also known as the carboxyl functional group. The carboxyl group is an ester where one substituent R is a hydrogen atom. The carboxyl group is usually denoted by -COOH.

The thiol functional group is similar to the hydroxyl group except the oxygen atom in the hydroxyl group is a sulfur atom in the thiol group. Thiol functional group is also known as a sulfhydryl functional group. Thiol functional groups have formula -SH.

The phenyl functional group is a common ring group. It is a benzene ring where one hydrogen atom is replaced by the R substituent group. Phenyl groups have formula C₆H₅.

In another particular embodiment, the linker region that connects the AML-specific antigen with at least one of the viral proteins is the result of the cross-linking of the AML-associated antigen and the VLP by a bifunctional cross-linking.

Types of bifunctional crosslinkers include homobifunctional crosslinkers, heterobifunctional crosslinkers, and photoreactive crosslinkers.

Homobifunctional crosslinkers are crosslinking agents that have the same functional chemistry at both ends of the structure, for example NH₂-NH₂, SH-SH, COOH-COOH. This comes with the added advantage of only needing to perform a one-step chemical crosslinking reaction in most cases. The most common homobifunctional crosslinkers are simple saturated carbon chains with two identical functional groups on each end, such as a diol or diamine.

Homobifunctional crosslinking reagents have identical reactive groups at either ends and are generally used in binding like functional groups. These reagents are mainly used to form intramolecular crosslinks and can be used in the preparation of polymers from monomers. Some common examples of amine-to-amine crosslinkers include disuccinimidyl suberate or DSS (ideal for receptor ligand cross-linking), disuccinimidyl tartrate or DST (used for applications wherein crosslink cleavability is required while keeping the protein disulfide bonds intact) and dithiobis succinimidyl propionate, or DSP (ideally used for crosslinking intracellular proteins prior to cell lysis and immunoprecipitation as well as for fixing protein interactions prior to identification of weak or transient protein interactions). Some common examples of sulfhydryl-to-sulfhydryl crosslinkers include BMOE and DTME.

Heterobifunctional crosslinkers are crosslinking agents that have different functional groups at each end of the structure, for example NH₂-SH, NH₂-COOH. This comes with the main advantage of more control over the synthesis through multi-step reactions. An example of a simple heterobifunctional crosslinker would be a carbon chain with a thiol on one end and an amine on the other.

Heterobifunctional crosslinking reagents possess two different reactive groups and can be used to link dissimilar functional groups. These reagents are used to produce multiple intermolecular crosslinks and conjugates using dissimilar biomolecules. Unlike homobifunctional crosslinkers that merely facilitates single-step conjugation of molecules, heterobifunctional crosslinkers allow for two-stage conjugations. This minimizes undesirable polymerization or self-conjugation. Some common examples of heterobifunctional crosslinkers include MDS (m-Maleimidobenzoyl-N-hydroxysuccinimide ester), GMBS (N-γ-Maleimidobutyryloxysuccinimide ester), EMCS (N-(ε-Maleimidocaproyloxy) succinimide ester), sulfo-EMCS (N-(ε-Maleimidocaproyloxy) sulfo succinimide ester), and sulfo-SMCC (Thermo 22322; MW 436.37, Spacer Arm 8.3 Å). The last one can link through one end an amino group (NH₂) and through the other end, a sulfhydryl group (SH).

Photoreactive crosslinkers have more complicated chemistry than the previously described types of crosslinker. These crosslinkers have photoactivatable functional groups that only become reactive when exposed to UV or visible light. These molecules typically use less common functional groups and more niche chemistry.

Photoreactive crosslinking reagents are used for non-specific bioconjugation and can be used to bind nucleic acids, proteins and other molecular structures. There are two photoreactive chemical groups that are being widely used in protein laboratories worldwide - aryl-azides and diazirines. Aryl azides (N-((2-pyridyldithio)ethyl)-4-azidosalicylamide) are the most widely used photoreactive reagents in crosslinking reactions. Upon exposure to 250-350nm UV light, these reagents can facilitate the formation of a nitrene group that may set off an addition reaction with the double bonds. Additionally, these crosslinkers may initiate the production of C-H insertion products or react with a nucleophile. Some common crosslinking reagents that belong to this group include ANB-NOS (N-5-Azido-2-nitrobenzyloxysuccinimide) and Sulfo-SANPAH.

On the other hand, NHS-ester diazirines or azipentanoates contain a photoactivatable diazirine ring and an N-hydroxysuccinimide (NHS) ester which efficiently reacts with primary amino groups in neutral to basic buffers (pH 7 to 9) to form stable amide bonds. It exhibits better photostability as compared to the phenyl azide group and can be easily activated with long-wave ultraviolet light (330 to 370nm) to produce carbene intermediates that form covalent bonds with any peptide backbones or amino acid side chains within the spacer arm distance.

### Location of the AML-specific antigen in the structural protein

In those embodiments wherein the AML-specific antigen is provided as a fusion protein with at least one of the viral structural proteins, the AML-specific antigen can be found in any position with respect to the structural protein, in particular TrV structural protein, to which it is fused. Thus, the invention contemplates a fusion protein comprising the TrV structural protein and the AML-specific antigen wherein the AML-specific antigen is fused to the C-terminus of the TrV structural protein. The inventions also contemplates a fusion protein comprising the TrV structural protein and the AML-specific antigen wherein the AML-specific antigen is fused to the N-terminus of the TrV structural protein. Thus, the invention contemplates a fusion protein comprising the TrV structural protein and the AML-specific antigen wherein the AML-specific antigen is inserted within the TrV structural protein.

In a particular embodiment, the AML-specific antigen is inserted inside the amino acid sequence of at least one viral structural protein, wherein said viral structural protein is TrV VP1, VP2, VP3, VP4 or VP0. The introduction of said AML-specific antigen in said at least one TrV structural protein must be performed such that it does not substantially alter the self-assembly capacity of said structural protein; and, furthermore, said AML-specific antigen must be located in a region of said capsid structural protein accessible to the solvent in the oligomeric structures formed after the self-assembly of the capsid.

In this particular embodiment, given that the AML-specific antigen is inserted inside the amino acid sequence of at least one TrV structural capsid protein, the fusion protein maintains the amino and carboxyl termini of said structural capsid protein and the AML-specific antigen is included inside the amino acid sequence of said structural capsid protein, for example, between two contiguous amino acids of said capsid protein or, alternatively, replacing one or more amino acids of said capsid protein. By way of illustration, in a particular embodiment, said AML-specific antigen is located between 2 contiguous amino acids of the structural capsid protein, i.e., between the "x" residue and the "x+1" residue of the amino acid sequence of said structural capsid protein; whereas, in another particular embodiment, due to the strategy followed for the introduction of the AML-specific antigen, one or more residues located in the regions adjacent to the point of insertion can be lost. Furthermore, given that the AML-specific antigen must be located in a region of said structural capsid protein accessible to the solvent in the VLPs formed according to the invention, the AML-specific antigen must be inserted in a suitable region. Particularly suitable regions of the structural protein for introducing the peptide insert are the loops present in said structural capsid proteins, particularly those loops which, in the oligomeric structures that they form, are exposed to the solvent.

To choose the suitable site of insertion of the AML-specific antigen of interest according to the invention in a TrV structural capsid protein it is convenient to know the atomic structure of the capsid protein to be genetically modified. The atomic structure of a structural protein can be determined by means of using X-ray diffraction. The use of suitable computer programs, such as the UCSF Chimera program (http://www.cgl.ucsf.edu/chimera/) for example, allows locating the regions of the protein under study which can potentially be genetically modified. These merely theoretical predictions initially serve to: i) discard regions that are not exposed to the solvent; and ii) identify regions the modification of which could cause a serious alteration in the secondary structure of the protein. Once the atomic structure of the capsid protein is known, a strategy can be designed to incorporate the peptide insert in the suitable site, for example, in a loop of said capsid protein which is exposed to the solvent when said structural protein is assembled with the rest of the structural proteins of the TrV and forms the oligomeric structure.

In a particular embodiment, said structural protein for the insertion of the AML-specific antigen of interest is selected from the group comprising VP1, VP2, VP3, VP4, VP0 and variants thereof. Sequences of VP1, VP2 and VP3 are located under Accession No. Q9QEY5 (UniProt database version 40, as of April 3, 2013, residues 1-255 corresponding to VP2, residues 313-597 corresponding to VP3 and residues 598-868 corresponding to VP1) and VP4 sequence is described in Agirre J et al. 2011 Virology 409: 91-101 (residues 256-312 in Q9QEY5). In this case, for the purpose of designing the strategy for introducing the AML-specific antigen in the suitable region, an in-depth study of the atomic structure of said TrV capsid proteins, which has already been described (Squires G *et al.* 2013 Acta Cryst D69: 1026-1037) can be followed.

In a particular embodiment, the AML-specific antigen of interest is inserted inside the amino acid sequence of at least one TrV structural protein, is bound to the amino terminal end of at least one TrV structural protein and/or is bound to the carboxy terminal end of at least one TrV structural protein wherein said at least one TrV structural protein is the same TrV structural protein or is a different TrV structural protein.

In a particular embodiment, regions for insertion/substitution in TrV structural proteins include the following regions:
1) in VP1 protein
   - from Ala681 to Trp688 according to Q9QEY5 Uniprot sequence (UniProt database version 40, as of April 3, 2013; see Figure1) (corresponding to Ala84 to Trp91 in VP1 sequence): stretch of 8 amino acid long located between two structural β-strands Pro 675-Tyr679 according to Q9QEY5 Uniprot sequence (Pro78-Tyr82 in VP1 sequence).
   - from Asp841 to Thr854 according to Q9QEY5 Uniprot sequence (Asp244 to Thr257 in VP1 sequence): stretch of 14 amino acids long located between two structural α-hehxes Ala836-Arg840 in Q9QEY5 (Ala239-Arg243 in VP1 sequence), and Phe855-Leu860 in Q9QEY5 (Phe258-Leu263 in VP1 sequence).
   - from Ser862 to Thr868 in Q9QEY5 sequence (Ser265 to Thr271 in VP1 sequence): unstructured stretch at the protein C-terminal end.
2) in VP2 protein:
   - from Leu1 to Asn14 (same as in Q9QEY5 numbering; see Figure1), the N-terminal which is disordered in the crystallographic structure.
   - from Thr97 to Lys103 according to Q9QEY5 Uniprot sequence: stretch between two structural β-strands (Gln88-Asn96, and Glu104-Leu110).
   - from Gly246 to Val253 according to Q9QEY5: C-terminal region pointing to the capsid exterior and after a β-strand (Ala230-Leu245 according to Q9QEY5).
3) in VP3 protein:
   - from Pro588 to Ile595 according to Q9QEY5 (Pro276 to Ile283 in VP3 sequence; see Figure1): C-terminal region located after a structural β-strand Pro580-Pro588 (Pro268-Pro276 in VP3 sequence), an aminoacid segment with unknown structure after Pro588 (Pro276 in VP3 sequence).
4) in VP4 protein:
   - from Lys258 to Leu310 according to Q9QEY5 (Lys3 to Leu55 in VP4 sequence; see Figure1).

Therefore, in a particular embodiment, the AML-specific antigen is located (numbers correspond to amino acids of protein precursor P1; see Figure 1):
- in VP1 structural protein, replacing region from Ala681 to Trp688, replacing the VP1 region from Asp841 to Thr854, and/or adding the epitope between Ser862 and Thr868 in VP1 protein, according to Q9QEY5 Uniprot sequence,
- in VP2 structural protein, replacing region from Leu1 to Asn14, and/or Thr97 to Lys103, and/or between Gly246 and Val253 in VP2 protein, according to Q9QEY5 Uniprot sequence,
- in VP3 structural protein, replacing region from Pro588 to Ile595, according to Q9QEY5 Uniprot sequence, and/or
- in VP4 in the region from Lys258 to Leu310 according to Q9QEY5.

In another particular embodiment, said AML-specific antigen is bound to the amino or carboxyl terminus of at least one TrV capsid structural protein. Therefore, in a particular embodiment, said AML-specific antigen is bound to the amino terminus of said TrV capsid protein, whereas, in another particular embodiment, said AML-specific antigen is bound to the carboxyl terminus of said TrV capsid protein.

In another particular embodiment, the TrV capsid protein comprises two AML-specific antigens bound to said protein, wherein each of said AML-specific antigen is bound to each of the amino and carboxyl termini of said capsid protein.

In another particular embodiment, the VLP of the invention comprises more than one AML-specific antigen, wherein said more than one AML-specific antigen are provided as fusion proteins with one or more TrV structural proteins or wherein said antigens are connected to one or more TrV structural proteins by a linker region.

### Polynucleotides of the invention, vectors and host cells

Another aspect of the invention relates to a polynucleotide (hereinafter the first polynucleotide of the invention) encoding a fusion protein selected from the group consisting of
(i) a fusion protein comprising a structural protein from a virus of the *Dicistroviridae* family selected from the group consisting of VP1, VP2, VP3, VP4 and VP0, or a functionally equivalent variant thereof, and an AML-specific antigen, or
(ii) a fusion protein comprising a structural protein precursor (P1) from a virus of the *Dicistroviridae* family, or a functionally equivalent variant thereof, and an AML-specific antigen.

In a preferred embodiment, the virus of the *Dicistroviridae* family is the *Triatoma virus* (TrV).

The structural proteins of viruses of the *Dicistroviridae* family, particularly TrV, have been previously described in the context of the VLP of the invention and include VP1, VP2, VP3, VP4 and VP0. In a particular embodiment, the polynucleotide of the invention encodes a fusion protein comprising the complete *Triatoma virus* structural protein precursor P1, or a functional equivalent variant thereof. TrV structural proteins precursor P1 is encoded by the TrV ORF2 viral genome (nucleotides 6109-8715, according to Czibener C et al. 2000 J Gen Virology 81: 1149-1154, and GenBank accession number AF178440).

In a more particular embodiment, the polynucleotide of the invention further comprises a nucleotide sequence encoding the non-structural protein NS of the *Triatoma virus* according to GenBank accession number AF178440 or a functionally equivalent variant thereof. TrV polyprotein precursor of non-structural proteins is encoded by the 5' ORF1 TrV viral genome (nucleotides 549-5933, according to Czibener C et al. 2000 J Gen Virology 81: 1149-1154, and GenBank accession number AF178440). TrV non-structural proteins include the viral polymerase, the viral protease and the viral helicase.

In a particular embodiment, the AML-specific antigen comprised by the fusion protein encoded by the first polynucleotide of the invention is inserted inside the amino acid sequence of at least one TrV structural protein, is bound to the amino terminal end of at least one TrV structural protein and/or is bound to the carboxy terminal end of at least one TrV structural protein wherein said at least TrV structural protein is the same TrV structural protein or is a different TrV structural protein.

In a particular embodiment, the AML-specific antigen comprised by the fusion protein encoded by the first polynucleotide of the invention is located at a position selected from the group consisting of (numbers correspond to amino acids of protein precursor P1; see Figure1):
- in VP1 structural protein, replacing region from Ala681 to Trp688,
- in VP1 structural protein, replacing region from Asp841 to Thr854
- in VP1 structural protein, after Ser862,
- in VP2 structural protein, replacing region from Leu1 to Asn14,
- in VP2 structural protein, replacing region from Thr97 to Lys103,
- in VP2 structural protein, replacing region from Gly246 and Val253,
- in VP3 structural protein, replacing region from Pro588 to Ile595,
- in VP4 in the region from Lys258 to Leu310.

AML-specific antigens according to the invention have been previously described in the context of the VLP of the invention. The AML-specific antigen is selected from the group consisting of: (i) an antigen derived from the nucleophosmin 1 protein, (ii) an antigen derived from the WT1 protein, (iii) an antigen derived from the isocitrate dehydrogenase 1 protein, and (iv) an antigen derived from a mutant FLT3 gene comprising an internal tandem duplication.

The AML-specific antigen comprises an epitope which is selected from the group consisting of the sequences AVEEVSLRK peptide (UniProt:E5RI98 and P06748) (SEQ ID NO: 1), the RMFPNAPYL peptide from Wilms tumor protein 1 (UniProt:P19544) (SEQ ID NO: 2), the GWVKPIIIGHHAYGDQYRAT peptide (IEDB (iedb.org) epitope ID 1445707) (SEQ ID NO: 3) or the YVDFREYEYY peptide (IEDB epitope ID 858644) (SEQ ID NO: 4).

Another aspect of the invention relates to a polynucleotide (hereinafter the second polynucleotide of the invention) encoding a fusion protein selected from the group consisting of
(i) a fusion protein comprising a structural protein from a virus of the *Dicistroviridae* family selected from the group consisting of VP1, VP2, VP3, VP4 and VP0, or a functionally equivalent variant thereof, and a first member of a binding pair, or
(ii) a structural protein precursor (P1) from a virus of the *Dicistroviridae* family, or a functionally equivalent variant thereof, and a first member of a binding pair.

In a particular embodiment, the virus of the *Dicistroviridae* family is the *Triatoma* virus (TrV).

Binding pairs according to the invention have been previously described in the context of the VLP of the invention. In a particular embodiment, the first member of the binding pair is selected from the group consisting of a peptide comprising the tripeptide Arg-Gly-Asp, a peptide comprising the sequence XBBXBX, wherein X represents any amino acid and B represents Arg or Lys, and a peptide comprising the sequence XBBBXXBX, wherein X represents any amino acid and B represents Arg or Lys and a biotin acceptor peptide (BAP).

In a more particular embodiment, the second polynucleotide of the invention further comprises a nucleotide sequence encoding the non-structural protein NS of the *Triatoma virus* according to GenBank accession number AF178440 or a functionally equivalent variant thereof.

In another particular embodiment, the second polynucleotide of the invention further comprises a nucleotide sequence encoding the structural protein precursor P1 according to GenBank accession number AF178440 or a functionally equivalent variant thereof.

The polynucleotides encoding a fusion protein comprising a structural protein from TrV and an AML-specific antigen or a first member of a binding pair according to the present invention (the first polynucleotide of the invention or the second polynucleotide of the invention) may be conveyed in a vector in order to allow adequate propagation of said polynucleotides. Therefore, the invention also contemplates that the polynucleotide encoding a fusion protein comprising a structural protein from the *Triatoma virus* and an AML-specific antigen or a first member of a binding pair, as described above is comprised by a vector (hereinafter the vector of the invention).

Thus, in another aspect, the invention relates to a vector comprising a polynucleotide encoding a fusion protein comprising a structural protein from the *Triatoma virus* and an AML-specific antigen or a first member of a binding pair. The first and the second polynucleotides of the invention have been described previously.

A person skilled in the art will understand that there is no limitation as regards the type of vector which can be used because said vector can be a cloning vector suitable for propagation and for obtaining the polynucleotides or suitable gene constructs or expression vectors in different heterologous organisms suitable for purifying the conjugates. Thus, suitable vectors according to the present invention include prokaryotic expression vectors (e.g. pUC18, pUC19, Bluescript and their derivatives), mp18, mp19, pBR322, pMB9, CoIEI, pCRI, RP4, phages and shuttle vectors (e.g. pSA3 and pAT28), yeast expression vectors (e.g. vectors of the type of 2 micron plasmids), integration plasmids, YEP vectors, centromeric plasmids and the like, insect cell expression vectors (e.g. the pAC series and pVL series vectors), plant expression vectors, such as vectors of expression in plants (e.g. pIBI, pEarleyGate, pAVA, pCAMBIA, pGSA, pGWB, pMDC, pMY, pORE series vectors), and eukaryotic expression vectors based on viral vectors (e.g. adenoviruses, viruses associated to adenoviruses as well as retroviruses and lentiviruses, baculovirus), as well as non-viral vectors (e.g. pSilencer 4.1-CMV (Ambion^{®}, Life Technologies Corp., Carlsbad, CA, US), pcDNA3, pcDNA3.1/hyg pHCMV/Zeo, pCR3.1, pEFI/His, pIND/GS, pRc/HCMV2, pSV40/Zeo2, pTRACER-HCMV, pUB6/V5-His, pVAXI, pZeoSV2, pCI, pSVL and pKSV-10, pBPV-1, pML2d and pTDTI). In a preferred embodiment, the vector of the invention is a baculovirus vector, more preferably the pFastBac1 vector.

Vectors may further contain one or more selectable marker sequences suitable for use in the identification of cells which have or have not been transformed or transfected with the vector. Markers include, for example, genes encoding proteins which increase or decrease either resistance or sensitivity to antibiotics or other compounds (e.g. hyg encoding hygromycin resistance (GenBank accession no. AF025746; or AF025747) and Kan^{R} (GenBank accession no. U75323), genes which encode enzymes whose activities are detectable by standard assays known in the art (e.g. β-galactosidase or luciferase), and genes which visibly affect the phenotype of transformed or transfected cells, hosts, colonies or plaques such as various fluorescent proteins (e.g. green fluorescent protein, GFP). Alternatively, the vectors of the present invention may carry a non-antibiotic selection marker, including, for instance, genes encoding a catabolic enzyme which enables the growth in medium containing a substrate of said catabolic enzyme as a carbon source. An example of such a catabolic enzyme includes, but is not restricted to, lacYZ encoding lactose uptake and beta-galactosidase (GenBank accession nos. J01636, J01637, K01483, or K01793). Other selection markers that provide a metabolic advantage in defined media include, but are not restricted to, galTK (GenBank accession no. X02306) for galactose utilization, sacPA (GenBank accession no. J03006) for sucrose utilization, trePAR (GenBank accession no. Z54245) for trehalose utilization and xylAB (GenBank accession nos. CAB 13644 and AAB41094) for xylose utilization. Alternatively, the selection can involve the use of antisense mRNA to inhibit a toxic allele, for instance the sacB allele (GenBank accession no. NP 391325).

Vectors according to the invention can also comprise at least one heterologous sequence encoding a cytokine, which is used to elicit augmented host responses to the immunogen vector. The particular cytokine encoded by the vector is not critical to the present invention includes, but not limited to, interleukin-4 (herein referred to as "IL-4"), IL-5, IL-6, IL-10, IL-12 p40, IL-12 p70, TGFβ and TNFα.

Vectors according to the invention can also comprise a promoter region, which can be used to regulate the transcription of the polynucleotides of the invention. The term "promoter", as used herein, refers to a nucleic acid fragment that functions to control the transcription of one or more genes, located upstream with respect to the direction of transcription of the transcription initiation site of the gene, and is structurally identified by the presence of a binding site for DNA- dependent RNA polymerase, transcription initiation sites and any other DNA sequences, including, but not limited to transcription factor binding sites, repressor and activator protein binding sites, and any other sequences of nucleotides known to one of skill in the art to act directly or indirectly to regulate the amount of transcription from the promoter.

Said promoter region can comprise a constitutive promoter, i.e., a promoter which directs the transcription in a basal manner, or an inducible promoter, in which the transcriptional activity requires an external signal. Suitable constitutive promoters for regulating the transcription are, among others, the CMV promoter, the SV40 promoter, the DHFR promoter, the mouse mammary tumor virus (MMTV) promoter, the elongation factor 1a (EFIa) promoter, the albumin promoter, the ApoA1 promoter, the keratin promoter, the CD3 promoter, the immunoglobulin heavy or light chain promoter, the neurofilament promoter, the neuron-specific enolase promoter, the L7 promoter, the CD2 promoter, the myosin light chain kinase promoter, the HOX gene promoter, the thymidine kinase promoter, the RNA Polymerase II promoter, the MyoD gene promoter, the phosphoglycerokinase (PGK) gene promoter, the low-density lipoprotein (LDL) promoter, the actin gene promoter. The inducible promoters which can be used in the context of the present invention are preferably those which respond to an inducing agent, which show nil or negligible basal expression in the absence of inducing agent and which are capable of promoting the activation of the gene located in the 3' position. Depending on the type of inducing agent, the inducible promoters are classified as Tet on/off promoters (Gossen, M. and H. Bujard (1992) Proc.Natl.Acad.Sci.USA, 89:5547-5551; Gossen, M. et al., 1995, Science 268:1766-1769; Rossi, F.M.V. and H.M. Blau, 1998, Curr. Opin. Biotechnol. 9:451-456); Pip on/off promoters (US6287813); antiprogestin-dependent promoters (US2004132086), ecdysone-dependent promoters (Christopherson et al., 1992, Proc.Natl.Acad.Sci.USA, 89:6314-6318; No et al., 1996, Proc.Natl.Acad.Sci.USA, 93:3346-3351, Suhr et al., 1998, Proc.Natl.Acad.Sci.USA, 95:7999-8004 and WO9738117), a metallothionein-dependent promoter (WO8604920) and rapamycin-dependent promoters (Rivera et al., 1996, Nat.Med. 2:1028-32).

Additionally, the vector can contain additional expression-regulating elements such as start and stop signals, cleavage sites, polyadenylation signal, origin of replication, transcriptional activators (enhancers), transcriptional silencers (silencers), etc. Generally, said elements are chosen depending on the host cells which are to be used.

The vector can additionally contain a transcription terminator sequence, such as the terminator of the bovine or human growth hormone gene, the terminator of the SV40 early genes, the terminators of the beta globin genes.

The polynucleotides and vectors according to the invention may be provided within host cells. Furthermore, the present invention also contemplates a host cell (hereinafter the host cell of the invention) comprising the polynucleotides of the invention encoding a fusion protein comprising a structural protein from a virus of the *Dicistroviridae* family, particularly the *Triatoma virus,* and a AML-specific antigen or a first member of a binding pair, as described above as well as a host cell comprising the vector of the invention comprising said polynucleotides as described above.

According to the invention, the host cell can be a bacterium, wherein said bacterium can be a gram negative bacterial cell, this term intended to include all facultative anaerobic Gram-negative cells of the family *Enterobacteriaceace* such as *Escherichia, Shigella, Citrobacter, Salmonella, Klebsiella, Enterobacter, Erwinia, Kluyvera, Serratia, Cedecea, Morganella, Hafhia, Edwardsiella, Providencia, Proteus* and *Yersinia.* In another preferred embodiment, the bacterium is a gram positive bacterial cell of the genus *Mycobacteriaceae* such as *M. bovis-BCG, M. leprae, M. marinum, M. smegmatis* and *M. tuberculosis.* In another embodiment, the host cell can be an insect cell including, without limitation, Sf9 cells, SF21 cells, SF+ cells, High-Five cells, or insect larval cells. In a particular embodiment, the host cell is an insect cell, more particularly a High Five (H5) (BTI-TN-5B1-4) insect cell, commercially available from, for example, Invitrogen.

The polynucleotide DNA of interest can be integrated or incorporated into the host cell genome and is referred to as integrated DNA or integrated DNA of interest. As a result, the DNA of interest can be introduced stably into and expressed in a host cell (i.e. production of foreign proteins is carried out from the DNA of interest present in the host cell). Alternatively, the DNA of interest is integrated into the host cell DNA, as a result of homologous recombination. A recombinant plasmid is used for introduction of the DNA of interest into the host cells and for stable integration of the DNA into the host cell genome. The recombinant plasmid used includes: 1) host cell sequences (referred to as plasmid-borne host cell sequences) necessary for homologous recombination to occur (between plasmid-borne mycobacterial sequences and sequences in the mycobacterial genome); 2) DNA sequences necessary for replication and selection; and 3) the DNA of interest (e.g. DNA encoding a selectable marker and DNA encoding a protein or polypeptide of interest). The recombinant plasmid is introduced, using known techniques, into the host cells.

### Processes for producing a virus-like particle expressing an AML-specific antigen of interest

The present invention relates to a process (hereinafter the first process of the invention) for obtaining the *Triatoma virus* (TrV) VLP modified to express an AML-specific antigen comprising the steps:
(i) contacting a host cell with a first polynucleotide which encodes a fusion protein comprising an AML-specific antigen and the structural protein precursor (P1) of TrV or a functionally equivalent variant thereof, wherein optionally the host cell is additionally contacting with a polynucleotide comprising a sequence encoding the non-structural protein precursors (NS) of TrV or a functionally equivalent variant thereof, under conditions suitable for the entry of said polynucleotide into the cell;
(ii) maintaining the cell under conditions suitable for the expression of the polynucleotide introduced in the cell in step (i), and
(iii) recovering the VLPs from the culture obtained.

Thus, in a first step of the first process of the invention, a host cell and a polynucleotide are contacted under conditions suitable for the entry of said polynucleotide into the cell, wherein said polynucleotide encodes a fusion protein comprising
(a) an AML-specific antigen of interest, and
(b) the structural protein precursor (P1) of the TrV or a functionally equivalent variant thereof.

Suitable polynucleotides for use in the first step have been described in the context of the polynucleotides of the invention and are equally useful for the methods for the generation of VLPs according to the present invention.

The conditions suitable for the entry of the polynucleotide in the host cell are well known for the person skilled in the art and include the microinjection of a composition comprising the polynucleotide or, alternatively, contacting the cell with a co-precipitate of the polynucleotide and calcium phosphate or calcium chloride or, alternatively, using transfection techniques such as DEAE-dextran, lipofection, electroporation as well as an entire series of transfection kits based on the previous techniques and which are commercially available. In a preferred embodiment, the first process of the invention comprises the use of transfection techniques.

Alternatively, the polynucleotide can form part of a viral vector, in which case the conditions suitable for the entry of the polynucleotide in the cell include contacting the cell with said viral vector. Viral vectors suitable for the introduction of the polynucleotide in the cell include but are not limited to adenoviral vectors, adeno-associated vectors, retroviral vectors, lentiviral vectors, baculovirus vectors, vectors based on herpes simplex or vectors based on alphaviruses. In a preferred embodiment of the invention, the polynucleotide is introduced into the cell using a vector, more preferably using a baculovirus vector.

The polynucleotide encoding a fusion protein comprising an AML-specific antigen of interest and the structural protein precursor (P1) of the *Triatoma virus* or a functionally equivalent variant thereof may further comprise additional elements for expression of the AML-specific antigen in an adequate host cell. Therefore, in a particular embodiment, the polynucleotide comprises a promoter region to regulate the transcription of said polynucleotide. Said promoter is selected from a constitutive promoter and an inducible promoter, which have been previously described in the context of the polynucleotide of the present invention. Additionally or alternatively, the polynucleotide comprises additional expression-regulating elements such as start and stop signals, cleavage sites, polyadenylation signal, origin of replication, transcriptional activators (enhancers), transcriptional silencers (silencers), transcription terminator sequences, etc. Generally, said elements are chosen depending on the host cells which are to be used.

The efficiency of the transfection will depend on a series of factors including the type of cell, the number of passages, the state of confluence as well as the transfection conditions (time, form of preparation of the liposomes or of the precipitates, etc.). All these parameters can be determined and adjusted by means of routine experimentation.

In a particular embodiment, the step (i) of the first process of the invention further comprises contacting the host cell used in said step (i) with one or more additional polynucleotides, under conditions suitable for the entry of said one or more additional polynucleotides into the cell, wherein said one or more additional polynucleotides comprise the nucleotide sequence encoding the non-structural (NS) protein precursor the *Triatoma virus* or a functionally equivalent variant thereof.

In a second step of the first process of the invention, the host cell is maintained under conditions suitable for the expression of the polynucleotide introduced in said cell in the previously described step (i) of the process.

Conditions suitable for the expression of the polynucleotide in the host cell include those conditions that allow the optimal growth of said host cell and those conditions that allow the protein expression in said host cell. Said culture conditions are typically different for each type of host cell. However, those conditions are known by skilled workers and are readily determined. Similarly, the duration of maintenance can differ with the host cells and with the amount of VLP desired to be prepared. Again, those conditions are well known and can readily be determined in specific situations. Additionally, specific culture conditions can be obtained from the examples herein.

According to the first process of the invention, the polynucleotide encoding a fusion protein comprising a AML-specific antigen and a the structural protein precursor (P1) of the *Triatoma* virus or a functionally equivalent variant and the polynucleotide which comprises a sequence encoding the non-structural protein precursor (NS) of the *Triatoma* virus or a functionally equivalent variant thereof are provided in the same polynucleotide or in different polynucleotides.

Therefore, in a particular embodiment, the first process of the invention comprises, in a first step, contacting a host cell with a polynucleotide under conditions suitable for the entry of said polynucleotide into the cell, wherein said polynucleotide encodes a fusion protein comprising
(a) an AML-specific antigen of interest, and
(b) the structural protein precursor (P1) of the *Triatoma virus* or a functionally equivalent variant thereof,
and wherein said polynucleotide further comprises a nucleotide sequence encoding the non-structural protein precursor (NS) of the *Triatoma virus* or a functionally equivalent variant thereof.

In an alternative embodiment, the first process of the invention comprises, in a first step, contacting a host cell with polynucleotides under conditions suitable for the entry of said polynucleotides into the cell, wherein a first polynucleotide encodes a fusion protein comprising
(a) an AML-specific antigen of interest, and
(b) the structural protein precursor (P1) of the *Triatoma virus* or a functionally equivalent variant thereof,
and wherein at least a second polynucleotide further comprises a nucleotide sequence encoding the non-structural protein precursor (NS) of the *Triatoma virus* or a functionally equivalent variant thereof.

In particular, sequences necessary for the generation of the VLPs by the process of the invention include the sequences encoding the structural capsid proteins (VP1, VP2, VP3 and VP4; or VP1, VP2 and VP0). Therefore, it is possible to contact the cell used in step (i) with polynucleotides encoding each of said structural proteins. However, the person skilled in the art will understand that use can be made of the genomic organization itself of the *Triatoma virus* wherein all the proteins necessary for the formation of the capsids are synthesized in the form of a polyprotein comprising both the aforementioned structural capsid proteins and the non-structural capsid proteins (structural protein precursor P1 and the non-structural NS protein precursor) but which are necessary for processing the polyprotein. In that case, the method of the invention would comprise contacting the cell with a polynucleotide comprising the sequence encoding said polyprotein.

The person skilled in the art will understand that the objective of steps (i) and (ii) of the first process of the invention is to provide the cell with all the components necessary for the formation of said VLPs, therefore they can be carried out sequentially or simultaneously.

The third step (iii) of the process is the recovering of the VLPs from the cell culture used in the previous steps. The particles can be recovered from the cells or from the supernatant of the culture medium. In the first case, the step of recovering requires the separation of the cells from the culture medium by any method known by the persons skilled in the art (trypsinization and centrifugation or filtration) and the rupture of said cells in an inert solution (freezing/thawing cycles, homogenization, sonication, cavitation, use of detergents and the like). Subsequently, it is possible to recover the particles from the cell homogenate or from the supernatant of the culture medium using well known methods such as density gradient ultracentrifugation (for example, using CsCI or sucrose) as has been described by Sanchez-Eugenia et al., J Gen Virol. 2015 Jan;96:64, Lombardo et al. (J. Virol., 1999, 73:6973-6983) and by Nick, H., et al. (J. Virol., 1976, 18:227-234), by means of chromatography, including affinity chromatography using anti-VP2 antibodies, by means of filtration using a series of fiberglass filters with a final pore size of 0.2 µm, as has been described in US2005214316.

According to the first process of the invention, the nucleotide sequence encoding the AML-specific antigen comprised by the fusion protein may be inserted in the nucleotide sequence encoding structural protein precursor P1 of the *Triatoma* virus or functionally equivalent variant thereof at different positions. In a particular embodiment, the AML-specific antigen is inserted in the sequence encoding P1 at
(i) a nucleotide position corresponding to an amino acid inside at least one TrV structural protein derived from P1,
(ii) a nucleotide position corresponding to the amino terminal end of at least one TrV structural protein derived from P1, and/or
(iii) a nucleotide position corresponding to the carboxy terminal end of at least one TrV structural protein derived from P1
wherein said at least one TrV structural protein is the same TrV structural protein or is a different TrV structural protein, and or wherein said AML-specific antigen is the same antigen or is a different antigen.

In another particular embodiment, the AML-specific antigen is located between nucleotide positions which result in a fusion protein wherein the AML-specific antigen is located at a position selected from the group consisting of (numbers correspond to amino acids of protein precursor P1; see Figure1):
- between amino acid positions Ala681 and Trp688 in VP1 structural protein,
- between amino acid positions Asp841 and Thr854 in VP1 structural protein,
- between amino acid positions Ser862 and Thr868 in VP1 structural protein,
- between amino acid positions Leu1 and Asn14 in VP2 structural protein,
- between amino acid positions Thr97 and Lys103 in VP2 structural protein,
- between amino acids positions Gly246 and Val253 in VP2 structural protein,
- between amino acids positions Pro588 to Ile595 in VP3 structural protein,
- between amino acids positions Lys258 to Leu310 in VP4 structural protein.

In another particular aspect, the invention relates to a process (hereinafter the second process of the invention) for obtaining a *Triatoma* virus (TrV) VLP modified to contain an AML-specific antigen comprising the steps:
(i) contacting a host cell with a first polynucleotide which encodes a fusion protein comprising a second member of a binding pair and the structural protein precursor (P1) of TrV or a functionally equivalent variant thereof, wherein optionally the host cell is additionally contacting with a polynucleotide comprising a sequence encoding the non-structural protein precursors (NS) of TrV or a functionally equivalent variant thereof, under conditions suitable for the entry of said polynucleotide into the cell;
(ii) maintaining the cell under conditions suitable for the expression of the polynucleotide introduced in the cell in step (i), and
(iii) recovering the VLPs from the culture obtained
(iv) contacting the VLPs recovered in step (iii) with the AML-specific antigen wherein the AML-specific antigen is provided as a fusion protein with a first member of a binding pair under conditions adequate for the interaction between said first and second members of the binding pair to occur.

Thus, in a first step of the second process of the invention, a host cell and a polynucleotide are contacted under conditions suitable for the entry of said polynucleotide into the cell, wherein said polynucleotide encodes a fusion protein comprising
(a) a second member of a binding pair, and
(b) the structural protein precursor (P1) of the TrV or a functionally equivalent variant thereof.

Suitable polynucleotides for use in the first step have been described in the context of the polynucleotides of the invention and are equally useful for the methods for the generation of VLPs according to the present invention.

The conditions suitable for the entry of the polynucleotide in the host cell are well known for the person skilled in the art and have been explained in the first process of the invention and are applicable to the second process of the invention. In a preferred embodiment, the second process of the invention comprises the use of transfection techniques.

The polynucleotide encoding a fusion protein comprising a second member of a binding pair and the structural protein precursor (P1) of the *Triatoma virus* or a functionally equivalent variant thereof may further comprise additional elements for the expression of the second member of a binding pair in an adequate host cell. Therefore, in a particular embodiment, the polynucleotide comprises a promoter region to regulate the transcription of said polynucleotide. Said promoter is selected from a constitutive promoter and an inducible promoter, which have been previously described in the context of the polynucleotide of the present invention. Additionally or alternatively, the polynucleotide comprises additional expression-regulating elements such as start and stop signals, cleavage sites, polyadenylation signal, origin of replication, transcriptional activators (enhancers), transcriptional silencers (silencers), transcription terminator sequences, etc. Generally, said elements are chosen depending on the host cells which are to be used.

The efficiency of the transfection will depend on a series of factors including the type of cell, the number of passages, the state of confluence as well as the transfection conditions (time, form of preparation of the liposomes or of the precipitates, etc.). All these parameters can be determined and adjusted by means of routine experimentation.

In a particular embodiment, the step (i) of the second process of the invention further comprises contacting the host cell used in said step (i) with one or more additional polynucleotides, under conditions suitable for the entry of said one or more additional polynucleotides into the cell, wherein said one or more additional polynucleotides comprise the nucleotide sequence encoding the non-structural (NS) protein precursor the *Triatoma virus* or a functionally equivalent variant thereof.

In a second step of the second process of the invention, the host cell is maintained under conditions suitable for the expression of the polynucleotide introduced in said cell in the previously described step (i) of the process. Conditions suitable for the expression of the polynucleotide in the host cell have been explained in the first process of the invention and are applicable to the second process of the invention.

According to the second process of the invention, the polynucleotide encoding a fusion protein comprising a second member of a binding pair and a the structural protein precursor (P1) of the *Triatoma* virus or a functionally equivalent variant and the polynucleotide which comprises a sequence encoding the non-structural protein precursor (NS) of the *Triatoma* virus or a functionally equivalent variant thereof are provided in the same polynucleotide or in different polynucleotides.

Therefore, in a particular embodiment, the second process of the invention comprises, in a first step, contacting a host cell with a polynucleotide under conditions suitable for the entry of said polynucleotide into the cell, wherein said polynucleotide encodes a fusion protein comprising
(a) a second member of a binding pair, and
(b) the structural protein precursor (P1) of the *Triatoma virus* or a functionally equivalent variant thereof,
and wherein said polynucleotide further comprises a nucleotide sequence encoding the non-structural protein precursor (NS) of the *Triatoma virus* or a functionally equivalent variant thereof.

In an alternative embodiment, the second process of the invention comprises, in a first step, contacting a host cell with polynucleotides under conditions suitable for the entry of said polynucleotides into the cell, wherein a first polynucleotide encodes a fusion protein comprising
(a) a second member of a binding pair, and
(b) the structural protein precursor (P1) of the *Triatoma virus* or a functionally equivalent variant thereof,
and wherein at least a second polynucleotide further comprises a nucleotide sequence encoding the non-structural protein precursor (NS) of the *Triatoma virus* or a functionally equivalent variant thereof.

The person skilled in the art will understand that the objective of steps (i) and (ii) of the second process of the invention is to provide the cell with all the components necessary for the formation of said VLPs, therefore they can be carried out sequentially or simultaneously.

The third step (iii) of the second process of the invention is the recovering of the VLPs from the cell culture used in the previous steps. The conditions of the recovering have been explained in the first process of the invention and are applicable to the second process of the invention.

In a fourth step of the second process of the invention, the VLPs recovered in step (iii) are contacted with the AML-specific antigen wherein the AML-specific antigen is provided as a fusion protein with a first member of a binding pair under conditions adequate for the interaction between said first and second members of the binding pair to occur. Conditions suitable for the interaction between said first and second members of the binding pair include those conditions that allow the specific interaction between the two groups. Those conditions are known by skilled workers and can readily be determined in specific situations.

According to the second process of the invention, the nucleotide sequence encoding the second member of the binding pair comprised by the fusion protein may be inserted in the nucleotide sequence encoding structural protein precursor P1 of the *Triatoma* virus or functionally equivalent variant thereof at different positions. In a particular embodiment, the second member of the binding pair is inserted in the sequence encoding P1 at
(i) a nucleotide position corresponding to an amino acid inside at least one TrV structural protein derived from P1,
(ii) a nucleotide position corresponding to the amino terminal end of at least one TrV structural protein derived from P1, and/or
(iii) a nucleotide position corresponding to the carboxy terminal end of at least one TrV structural protein derived from P1,
wherein said at least one TrV structural protein is the same TrV structural protein or is a different TrV structural protein, and or wherein said AML-specific antigen is the same antigen or is a different antigen.

In a particular embodiment, the second member of the binding pair is located between nucleotide positions which result in a fusion protein wherein the AML-specific antigen is located at a position selected from the group consisting of (numbers correspond to amino acids of protein precursor P1; see Figure1):
- between amino acid positions Ala681 and Trp688 in VP1 structural protein,
- between amino acid positions Asp841 and Thr854 in VP1 structural protein,
- between amino acid positions Ser862 and Thr868 in VP1 structural protein,
- between amino acid positions Leu1 and Asn14 in VP2 structural protein,
- between amino acid positions Thr97 and Lys103 in VP2 structural protein,
- between amino acids positions Gly246 and Val253 in VP2 structural protein,
- between amino acids positions Pro588 to Ile595 in VP3 structural protein,
- between amino acids positions Lys258 to Leu310 in VP4 structural protein.

In a particular embodiment, the first member of a binding pair is selected from the group consisting of a peptide comprising the tripeptide Arg-Gly-Asp, a peptide comprising the sequence XBBXBX, wherein X represents any amino acid and B represents Arg or Lys, and a peptide comprising the sequence XBBBXXBX, wherein X represents any amino acid and B represents Arg or Lys and a biotin acceptor peptide (BAP).

In a preferred embodiment, the first member of the binding pair is the biotinylated BAP and the second member of the binding pair is a molecule comprising a biotin-binding region.

In a more preferred embodiment, the molecule comprising a biotin-binding region is selected from the group comprising avidin, an avidin analogue, streptavidin, and streptavidin analogue.

In another aspect, the present invention relates to a process (hereinafter the third process of the invention) for obtaining a *Triatoma virus* (TrV) VLP modified to contain an AML-specific antigen comprising the steps:
(i) contacting a host cell with a first polynucleotide which encodes the structural protein precursor (P1) of TrV or a functionally equivalent variant thereof, wherein optionally the host cell is additionally contacting with a polynucleotide comprising a sequence encoding the non-structural protein precursors (NS) of TrV or a functionally equivalent variant thereof, under conditions suitable for the entry of said polynucleotide into the cell;
(ii) maintaining the cell under conditions suitable for the expression of the polynucleotide introduced in the cell in step (i), and
(iii) recovering the VLPs from the culture obtained
(iv) treating the VLPs obtained in step (iii) with a reagent that creates functional groups on the VLP thereby creating functionalized VLPs and reacting the functionalized VLPs with a functionalized form of the AML-specific antigen which contains functional groups which are reactive with the functional groups present in the functionalized VLPs or, alternatively, contacting the VLPs obtained in step (iii) with the AML-specific antigen in the presence of a bifunctional reagent that is capable of reacting with groups present in the VLP and in the AML-specific antigen thereby cross-linking the VLP and the antigen.

Thus, in a first step of the third process of the invention, a host cell and a polynucleotide are contacted under conditions suitable for the entry of said polynucleotide into the cell, wherein said polynucleotide encodes the structural protein precursor (P1) of the TrV or a functionally equivalent variant thereof.

Suitable polynucleotides for use in the first step have been described in the context of the polynucleotides of the invention and are equally useful for the methods for the generation of VLPs according to the present invention.

The conditions suitable for the entry of the polynucleotide in the host cell are well known for the person skilled in the art and have been explained in the first process of the invention and are applicable to the third process of the invention. In a preferred embodiment, the third process of the invention comprises the use of transfection techniques.

The polynucleotide encoding a fusion protein comprising the structural protein precursor (P1) of the *Triatoma virus* or a functionally equivalent variant thereof may further comprise additional elements. Therefore, in a particular embodiment, the polynucleotide comprises a promoter region to regulate the transcription of said polynucleotide. Said promoter is selected from a constitutive promoter and an inducible promoter, which have been previously described in the context of the polynucleotide of the present invention. Additionally or alternatively, the polynucleotide comprises additional expression-regulating elements such as start and stop signals, cleavage sites, polyadenylation signal, origin of replication, transcriptional activators (enhancers), transcriptional silencers (silencers), transcription terminator sequences, etc. Generally, said elements are chosen depending on the host cells which are to be used.

The efficiency of the transfection will depend on a series of factors including the type of cell, the number of passages, the state of confluence as well as the transfection conditions (time, form of preparation of the liposomes or of the precipitates, etc.). All these parameters can be determined and adjusted by means of routine experimentation.

In a particular embodiment, the step (i) of the third process of the invention further comprises contacting the host cell used in said step (i) with one or more additional polynucleotides, under conditions suitable for the entry of said one or more additional polynucleotides into the cell, wherein said one or more additional polynucleotides comprise the nucleotide sequence encoding the non-structural (NS) protein precursor the *Triatoma virus* or a functionally equivalent variant thereof.

In a second step of the third process of the invention, the host cell is maintained under conditions suitable for the expression of the polynucleotide introduced in said cell in the previously described step (i) of the process. Conditions suitable for the expression of the polynucleotide in the host cell have been explained in the first process of the invention and are applicable to the third process of the invention.

According to the third process of the invention, the polynucleotide encoding a fusion protein comprising the structural protein precursor (P1) of the *Triatoma* virus or a functionally equivalent variant and the polynucleotide which comprises a sequence encoding the non-structural protein precursor (NS) of the *Triatoma* virus or a functionally equivalent variant thereof are provided in the same polynucleotide or in different polynucleotides.

Therefore, in a particular embodiment, the third process of the invention comprises, in a first step, contacting a host cell with a polynucleotide under conditions suitable for the entry of said polynucleotide into the cell, wherein said polynucleotide encodes a fusion protein comprising the structural protein precursor (P1) of the *Triatoma virus* or a functionally equivalent variant thereof, and wherein said polynucleotide further comprises a nucleotide sequence encoding the non-structural protein precursor (NS) of the *Triatoma virus* or a functionally equivalent variant thereof.

In an alternative embodiment, the third process of the invention comprises, in a first step, contacting a host cell with polynucleotides under conditions suitable for the entry of said polynucleotides into the cell, wherein a first polynucleotide encodes a fusion protein comprising the structural protein precursor (P1) of the *Triatoma virus* or a functionally equivalent variant thereof, and wherein at least a second polynucleotide further comprises a nucleotide sequence encoding the non-structural protein precursor (NS) of the *Triatoma virus* or a functionally equivalent variant thereof.

The person skilled in the art will understand that the objective of steps (i) and (ii) of the third process of the invention, is to provide the cell with all the components necessary for the formation of said VLPs, therefore they can be carried out sequentially or simultaneously.

The third step (iii) of the third process of the invention is the recovering of the VLPs from the cell culture used in the previous steps. The conditions of the recovering have been explained in the first process of the invention and are applicable to the third process of the invention.

In a fourth step of the third process of the invention, the VLPs recovered in step (iii) are treated with a reagent that creates functional groups on the VLP thereby creating functionalized VLPs and reacting the functionalized VLPs with a functionalized form of the AML- specific antigen which functional groups which are reactive with the functional groups present in the functionalized VLPs. Alternatively, the VLPs are contacted with the AML-specific antigen in the presence of a bifunctional reagent that is capable of reacting with groups present in the VLP and in the AML-specific antigen thereby cross-linking the VLP and the antigen. Suitable functional groups have been described in the context of the VLPs of the invention and are equally useful for the third method of the invention. Suitable bifunctional reagents have been described in the context of the VLPs of the invention and are equally useful for the third method of the invention. Conditions suitable for the creation of functional groups and for the reaction between the two functional groups or the cross-linking are known by skilled workers and can readily be determined in specific situations.

In a particular embodiment of the processes of the invention (first, second and third processes of the invention), the AML-specific antigen is selected from the group consisting of:
(i) an antigen derived from the nucleophosmin 1 protein,
(ii) an antigen derived from the WT1 protein,
(iii) an antigen derived from the isocitrate dehydrogenase 1 protein, and
(iv) an antigen derived from a mutant FLT3 gene comprising an internal tandem duplication.

In another particular embodiment, the AML-specific antigen comprises an epitope which is selected from the group consisting of the sequences AVEEVSLRK peptide (UniProt:E5RI98 and P06748) (SEQ ID NO: 1), the RMFPNAPYL peptide from Wilms tumor protein 1 (UniProt:P19544) (SEQ ID NO: 2), the GWVKPIIIGHHAYGDQYRAT peptide (IEDB (iedb.org) epitope ID 1445707) (SEQ ID NO: 3) or the YVDFREYEYY peptide (IEDB epitope ID 858644) (SEQ ID NO: 4).

The present invention relates as well to the VLPs obtained by the first process of the invention, the second process of the invention and/or the third process of the invention.

The present invention relates as well to vaccine or immunogenic compositions comprising the VLPs as above, wherein said VLPs have been obtained by any of the processes of the invention as previously described.

### Therapeutic methods

The VLPs of the invention are particularly useful in the generation of an immune response against the AML-specific antigen comprised by said VLPs. Therefore, in another aspect, the present invention relates to a vaccine or immunogenic composition comprising a VLP of the invention as described above, the polynucleotides of the invention as described above, or a vector of the invention as described above. The pharmaceutical compositions according to the present invention comprise vaccines and immunogenic compositions containing the VLPs, the polynucleotides, or the vector of the invention.

In a particular embodiment, said pharmaceutical composition comprises a VLP according to the invention, wherein said VLP comprises an AML-specific antigen in such a way that an immune response against said AML-specific antigen is induced. In another particular embodiment, said pharmaceutical composition comprises a polynucleotide according to the invention, wherein said polynucleotide encodes a fusion protein comprising an AML-specific antigen in such a way that an immune response against said AML-specific antigen is induced. In another particular embodiment, said pharmaceutical composition comprises a vector according to the invention, wherein said vector encodes a fusion protein comprising an AML-specific antigen in such a way that an immune response against said AML-specific antigen is induced.

The vaccines and compositions are administered in a manner compatible with the dosage formulation, and in such amount as will be therapeutically effective and immunogenic. The quantity to be administered depends on the subject to be treated, capacity of the subject's immune system to generate a cellular immune response, and degree of protection desired. Precise amounts of active ingredient required to be administered depend on the judgment of the practitioner and are peculiar to each individual. However, suitable dosage ranges are of the order of about one microgram to about one milligram, preferably about 1 microgram and more preferably about 5 micrograms, and more preferably 100 micrograms active ingredient per kilogram bodyweight individual. Suitable regimes for initial administration and booster shots are also variable, but are typified by an initial administration followed in one or two week intervals by a subsequent injection or other administration.

The vaccines of the invention may be specially formulated for administration in solid or liquid form, including those adapted for the following: (a) oral administration, such as drenches (aqueous or non-aqueous solutions or suspensions), tablets, boluses, powders, granules, pastes, mouthwash or hydrogels, (b) parenteral administration, for instance, by subcutaneous, intramuscular or intravenous injection of, for example, a sterile solution or suspension, (c) intracavity administration (e.g. intraperitoneal instillation), intravesical (i.e. urinary bladder) instillation, (d) intraorgan administration (e.g. intraprostatical administration), (e) topical application (i.e. cream, ointment or spray applied to the skin), (f) intravaginal or intrarectal administration (e.g. as a pessary, cream, foam, enema or suppository) or (g) aerosol (e.g. as an aqueous aerosol, liposomal preparation or solid particles containing the agent (s)).

*In vitro* assays and animal studies can be used to determine an effective amount of the VLPs, polynucleotides or vectors of the invention, or combinations thereof. A person of ordinary skill in the art would select an appropriate amount of each individual compound in the combination for use in the aforementioned assays or similar assays. Changes in cell activity or cell proliferation can be used to determine whether the selected amounts are an effective amount for the particular combination of compounds. The regimen of administration also can affect what constitutes an effective amount. Further, several divided dosages, as well as staggered dosages, can be administered daily or sequentially, or the dose can be proportionally increased or decreased as indicated by the exigencies of the therapeutic situation.

In a particular embodiment, the VLPs, the polynucleotides or the vector of the present invention are used for the treatment of AML.

The amount of the VLPs, the polynucleotides or the vector of the present invention to be administered will vary depending on the species of the subject, as well as the type of AML. Preferably the subject is a mammal. More preferably, the subject is a human.

In another particular embodiment, the subject to which the VLPs, the polynucleotides or the vector, and pharmaceutical compositions thereof of the present invention are administered is a non-human mammal. In a preferred embodiment, the subject is a non-human primate, cow, goat, cat, dog, pig, buffalo, badger, possum, deer, or bison.

As mentioned above, the French-American-British (FAB) classification system divides AML into eight subtypes, M0 through to M7, based on the type of cell from which the leukemia developed and its degree of maturity. AML of types M0 to M2 may be called acute myeloblastic leukemia. Classification is done by examining the appearance of the malignant cells with light microscopy and/or by using cytogenetics to characterize any underlying chromosomal abnormalities. The subtypes have varying prognoses and responses to therapy.

The subtype M0 refers to acute myeloblastic leukemia, minimally differentiated. The subtype M1 refers to acute myeloblastic leukemia, without maturation. The subtype M2 refers to acute myeloblastic leukemia, with granulocytic maturation. The subtype M3 refers to promyelocytic, or acute promyelocytic leukemia (APL). The subtype M4 refers to acute myelomonocytic leukemia. The subtype M4eo refers to myelomonocytic together with bone marrow eosinophilia. The subtype M5 refers to acute monoblastic leukemia (M5a) or acute monocytic leukemia (M5b). The subtype M6 refers to acute erythroid leukemias, including erythroleukemia (M6a) and very rare pure erythroid leukemia (M6b). The subtype M7 refers to acute megakaryoblastic leukemia.

In a particular embodiment, the VLPs, the polynucleotides or the vectors of the present invention are for use in the treatment of the AML subtypes M0, M1, M2, M3, M4, M4eo, M5, M6 and/or M7.

While the terminology of the FAB system is still sometimes used, and it remains a valuable diagnostic tool in areas without access to genetic testing, this system has largely become obsolete in favour of the WHO classification, which correlates more strongly with treatment outcomes.

The WHO classification, which is based on a combination of genetic and immunophenotypic markers and morphology, defines the subtypes of AML and related neoplasms as:
(i) Acute myeloid leukemia with recurrent genetic abnormalities:
   - AML with translocations between chromosome 8 and 21 - [t(8;21)(q22;q22.1);] RUNX1-RUNX1T1; (ICD-O 9896/3);
   - AML with inversions in chromosome 16 - [inv(16)(p13.1q22)] or internal translocations in it - [t(16;16)(p13.1;q22);] CBFB-MYH11; (ICD-O 9871/3);
   - Acute promyelocytic leukemia with PML-RARA; (ICD-O 9866/3);
   - AML with translocations between chromosome 9 and 11 - [t(9;11)(p21.3;q23.3);] KMT2A-MLLT3;
   - AML with translocations between chromosome 6 and 9 - [t(6;9)(p23;q34.1);] DEK-NUP214;
   - AML with inversions in chromosome 3 - [inv(3)(q21.3q26.2)] or internal translocations in it - [t(3;3)(q21.3;q26.2);] GATA2, MECOM
   - Megakaryoblastic AML with translocations between chromosome 1 and 22 - [t(1;22)(p13.3;q13.1);] RBM15-MKL1;
   - AML with mutated NPM1
   - AML with biallelic mutations of CEBPA
   - AML with BCR-ABL1 and AML with mutated RUNX1
(ii) AML with myelodysplasia-related changes. This category includes people who have had a prior documented myelodysplastic syndrome (MDS) or myeloproliferative disease (MPD) that then has transformed into AML; who have cytogenetic abnormalities characteristic for this type of AML (with previous history of MDS or MPD that has gone unnoticed in the past, but the cytogenetics is still suggestive of MDS/MPD history); or who have AML with morphologic features of myelodysplasia (dysplastic changes in multiple cell lines). Cytogenetic markers for AML with myelodysplasia-related changes include:
   - Complex karyotype (meaning more than three chromosomal abnormalities)
   - Unbalanced abnormalities
      - Deletions or loss of chromosome 7 - [del(7q)/-7;]
      - Deletions or translocations in chromosome 5 - [del(5q)/t(5q);]
      - Unbalanced chromosomal aberrations in chromosome 17 - [i(17q)/t(17p);]
      - Deletions or loss of chromosome 13 - [del(13q)/-13;]
      - Deletions of chromosome 11 - [del(11q);]
      - Unbalanced chromosomal aberrations in chromosome 12 - [del(12p)/t(12p);]
      - Aberrations in chromosome X - [idic(X)(q13);]
   - Balanced abnormalities
      - Translocations between chromosome 11 and 16 - [t(11;16)(q23.3;q13.3);]
      - Translocations between chromosome 3 and 21 - [t(3;21)(q26.2;q22.1);]
      - Translocations between chromosome 1 and 3 - [t(1;3)(p36.3;q21.2);]
      - Translocations between chromosome 2 and 11 - [t(2;11)(p21;q23.3);]
      - Translocations between chromosome 5 and 12 - [t(5;12)(q32;p13.2);]
      - Translocations between chromosome 5 and 7 - [t(5;7)(q32;q11.2);]
      - Translocations between chromosome 5 and 17 - [t(5;17)(q32;p13.2);]
      - Translocations between chromosome 5 and 10 - [t(5;10)(q32;q21);]
      - Translocations between chromosome 3 and 5 - [t(3;5)(q25.3;q35.1);]
(iii) Therapy-related myeloid neoplasms. This category includes people who have had prior chemotherapy and/or radiation and subsequently develop AML or MDS. These leukemias may be characterized by specific chromosomal abnormalities, and often carry a worse prognosis.
(iv) Myeloid sarcoma. This category includes myeloid sarcoma, which is an extramedullary tumor of immature granulocytic cells.
(v) Myeloid proliferations related to Down syndrome. This category includes "transient abnormal myelopoiesis" and "myeloid leukemia associated with Down syndrome". In young children, myeloid leukemia associated with Down syndrome has a much better prognosis than other types of childhood AML. The prognosis in older children is similar to conventional AML.
(vi) AML not otherwise categorized. Includes subtypes of AML that do not fall into the above categories:
   - AML with minimal differentiation
   - AML without maturation
   - AML with maturation
   - Acute myelomonocytic leukemia
   - Acute monoblastic and monocytic leukemia
   - Pure erythroid leukemia
   - Acute megakaryoblastic leukemia
   - Acute basophilic leukemia
   - Acute panmyelosis with myelofibrosis

In a particular embodiment, the VLPs, the polynucleotides or the vectors of the present invention are for use in the treatment of the AML subtypes: acute myeloid leukemia with recurrent genetic abnormalities, AML with myelodysplasia-related changes, therapy-related myeloid neoplasms, myeloid sarcoma, myeloid proliferations related to Down syndrome, AML with minimal differentiation, AML without maturation, AML with maturation, acute myelomonocytic leukemia, acute monoblastic and monocytic leukemia, pure erythroid leukemia, acute megakaryoblastic leukaemia, acute basophilic leukaemia and/or acute panmyelosis with myelofibrosis.

In a preferred embodiment, the VLPs, the polynucleotides or the vectors of the present invention are for use in the treatment of the AML subtype characterized by presence of recurrent mutation of nucleophosmin-1 (NPM1).

In another preferred embodiment, the VLPs, the polynucleotides or the vectors of the present invention are for use in the treatment of the AML subtype characterized by presence of recurrent mutation of Wilms tumor protein 1 (WT1).

In another preferred embodiment, the VLPs, the polynucleotides or the vectors of the present invention are for use in the treatment of the AML subtype characterized by presence of recurrent mutation of isocitrate dehydrogenase (IDH).

In another preferred embodiment, the VLPs, the polynucleotides or the vectors of the present invention are for use in the treatment of the AML subtype characterized by presence of recurrent mutation of internal tandem repetitions of FLT3 (FLT3-ITD).

In further aspects, the present invention relates to:
1. A virus-like particle (VLP) comprising
   (i) the viral structural proteins VP1, VP2, VP3 and VP4, or the viral structural proteins VP1, VP2, and VP0, or the structural protein precursor (P1) of *Triatoma virus* (TrV), or a functionally equivalent variant of any of the above , and
   (ii) an acute myeloid leukaemia (AML) specific antigen
   wherein said antigen is provided as a fusion protein with at least one of said viral structural proteins or wherein said antigen is connected to at least one of the said viral proteins by a linker region.
2. The VLP according to aspect 1, wherein the AML-specific antigen is selected from the group consisting of:
   (i) an antigen derived from the nucleophosmin 1 protein,
   (ii) an antigen derived from the WT1 protein,
   (iii) an antigen derived from the isocitrate dehydrogenase 1 protein, and
   (iv) an antigen derived from a mutant FLT3 gene comprising an internal tandem duplication.
3. The VLP according to aspect 2, wherein the AML-specific antigen comprises an epitope which is selected from the group consisting of the sequences AVEEVSLRK peptide (UniProt:E5RI98 and P06748)(SEQ ID NO:1), the RMFPNAPYL peptide from Wilms tumor protein 1 (UniProt:P19544) (SEQ ID NO: 2), the GWVKPIIIGHHAYGDQYRAT peptide (iedb.org IEDB epitope ID 1445707) (SEQ ID NO: 3) or the YVDFREYEYY peptide (IEDB epitope ID 858644) (SEQ ID NO: 4).
4. The VLP according to any of aspects 1 to 3, wherein the linker region that connects the AML-specific antigen with at least one of the viral proteins is the result of the reaction between a first member of a binding pair and a second member of the binding pair.
5. The VLP according to any of aspects 1 to 4, wherein the AML-specific antigen comprises a first member of a binding pair.
6. The VLP according to aspect 5, wherein the first member of a binding pair is selected from the group consisting of a biotin acceptor peptide (BAP), a peptide comprising the tripeptide Arg-Gly-Asp, a peptide comprising the sequence XBBXBX, wherein X represents any amino acid and B represents Arg or Lys, and a peptide comprising the sequence XBBBXXBX, wherein X represents any amino acid and B represents Arg or Lys.
7. The VLP according to aspects 5 or 6, wherein the VLP is bound to a second member of a binding pair, said second member of the binding pair being bound to the first member of the binding pair.
8. The VLP according to aspect 7, wherein the first member of the binding pair is the biotinylated BAP and the second member of the binding pair is a molecule comprising a biotin-binding region.
9. The VLP according to aspect 8, wherein the molecule comprising a biotin-binding region is selected from the group comprising avidin, an avidin analog, streptavidin, and streptavidin analog.
10. The VLP according to any of aspects 1 to 3, wherein the linker region that connects the AML-specific antigen with at least one of the viral proteins is the result of the reaction between a first functional groups found within the VLP and a second functional group found in the AML-specific antigen or the result of the cross-linking of the AML-associated antigen and the VLP by a bifunctional cross-linking reagent.
11. The VLP according to any of aspects 1 to 10, wherein the AML-specific antigen is inserted inside the amino acid sequence of at least one TrV structural protein, is bound to the amino terminal end of at least one TrV structural protein and/or is bound to the carboxy terminal end of at least one TrV structural protein, said at least one TrV structural protein is the same TrV structural protein or is a different TrV structural protein.
12. The VLP according to aspect 11, wherein the AML-specific antigen is located (numbers correspond to amino acids of protein precursor P1)
   - between amino acid positions Ala681 and Trp688 in VP1 structural protein,
   - between amino acid positions Asp841 and Thr854 in VP1 structural protein,
   - between amino acid positions Ser862 and Thr868 in VP1 structural protein,
   - between amino acid positions Leu1 and Asn14 in VP2 structural protein,
   - between amino acid positions Thr97 and Lys103 in VP2 structural protein,
   - between amino acids positions Gly246 and Val253 in VP2 structural protein,
   - between amino acids positions Pro588 to Ile595 in VP3 structural protein,
   - between amino acids positions Lys258 to Leu310 in VP4 structural protein.
13. The VLP according to any of aspects 1 to 12, wherein the VLP comprises more than one AML-specific antigen, which may be the same or different and wherein each of said antigens are provided as fusion proteins with one or more TrV structural proteins or wherein said antigens are connected to one or more TrV structural proteins by a linker region.
14. The VLP according to any of aspects 1 to 13, wherein the AML-specific antigen is an antigen derived from the nucleophosmin 1 protein and wherein said antigen comprises the epitope with the sequence AVEEVSLRK peptide (UniProt:E5RI98 and P06748) (SEQ ID NO: 1).
15. A polynucleotide encoding a fusion protein selected from the group consisting of
   (i) a fusion protein comprising a structural protein from a virus of the *Dicistroviridae* family selected from the group consisting of VP1, VP2, VP3, VP4 and VP0, or a functionally equivalent variant thereof, and an AML-specific antigen, or
   (ii) a fusion protein comprising a structural protein precursor (P1) from a virus of the *Dicistroviridae* family, or a functionally equivalent variant thereof, and an AML-specific antigen.
16. The polynucleotide according to aspect 15, wherein the fusion protein further comprises a non-structural protein (NS) from a virus of the *Dicistroviridae* family, or a functionally equivalent variant thereof.
17. The polynucleotide according to any of aspects 15 or 16, wherein the virus of the *Dicistroviridae* family is the *Triatoma virus* (TrV).
18. The polynucleotide according to aspect 17, wherein the fusion protein comprises the complete *Triatoma virus* structural protein precursor P1 according to GenBank accession number AF178440 or a functionally equivalent variant thereof.
19. The polynucleotide according to aspect 18, wherein the fusion protein further comprises the non-structural protein NS of the *Triatoma virus* according to GenBank accession number AF178440 or a functionally equivalent variant thereof.
20. The polynucleotide according to any of aspects 15 to 19, wherein the AML-specific antigen is located at a position selected from the group consisting of (numbers correspond to amino acids of protein precursor P1):
   - between amino acid positions Ala681 and Trp688 in VP1 structural protein,
   - between amino acid positions Asp841 and Thr854 in VP1 structural protein,
   - between amino acid positions Ser862 and Thr868 in VP1 structural protein,
   - between amino acid positions Leu1 and Asn14 in VP2 structural protein,
   - between amino acid positions Thr97 and Lys103 in VP2 structural protein,
   - between amino acids positions Gly246 and Val253 in VP2 structural protein,
   - between amino acids positions Pro588 to Ile595 in VP3 structural protein,
   - between amino acids positions Lys258 to Leu310 in VP4 structural protein.
21. The polynucleotide according to any of aspects 15 to 20, wherein the AML-specific antigen is selected from the group consisting of:
   (i) an antigen derived from the nucleophosmin 1 protein,
   (ii) an antigen derived from the WT1 protein,
   (iii) an antigen derived from the isocitrate dehydrogenase 1 protein, and
   (iv) an antigen derived from a mutant FLT3 gene comprising an internal tandem duplication.
22. The polynucleotide according to any of aspects 15 to 21, wherein the AML-specific antigen comprises an epitope which is selected from the group consisting of the sequences AVEEVSLRK peptide (UniProt:E5RI98 and P06748) (SEQ ID NO: 1), the RMFPNAPYL peptide from Wilms tumor protein 1 (UniProt:P19544) (SEQ ID NO: 2), the GWVKPIIIGHHAYGDQYRAT peptide (IEDB (iedb.org) epitope ID 1445707) (SEQ ID NO: 3) or the YVDFREYEYY peptide (IEDB epitope ID 858644) (SEQ ID NO: 4).
23. A polynucleotide encoding a fusion protein selected from the group consisting of:
   (i) a fusion protein comprising a structural protein from a virus of the *Dicistroviridae* family selected from the group consisting of VP1, VP2, VP3, VP4 and VP0, or a functionally equivalent variant thereof, and a first member of a binding pair, or
   (ii) a structural protein precursor (P1) from a virus of the *Dicistroviridae* family, or a functionally equivalent variant thereof, and a first member of a binding pair.
24. The polynucleotide according to aspect 23, wherein the first member of the binding pair is selected from the group consisting of a peptide comprising the tripeptide Arg-Gly-Asp, a peptide comprising the sequence XBBXBX, wherein X represents any amino acid and B represents Arg or Lys, and a peptide comprising the sequence XBBBXXBX, wherein X represents any amino acid and B represents Arg or Lys and a biotin acceptor peptide (BAP).
25. The polynucleotide according to any of aspects 23 or 24, wherein the fusion protein further comprises a non-structural protein (NS) from a virus of the *Dicistroviridae* family, or a functionally equivalent variant thereof.
26. The polynucleotide according to any of aspects 23 to 25, wherein the virus of the *Dicistroviridae* family is the *Triatoma virus* (TrV).
27. The polynucleotide according to aspect 26, wherein the fusion protein comprises the complete *Triatoma virus* structural protein precursor P1 according to GenBank accession number AF178440 or a functionally equivalent variant thereof.
28. A vector comprising a polynucleotide according to any one of aspects 15 to 27.
29. A host cell comprising a polynucleotide according to any one of aspects 15 to 27 or a vector according to aspect 28.
30. A process for obtaining the *Triatoma virus* (TrV) VLP modified to express an AML-specific antigen comprising the steps:
   (i) contacting a host cell with a first polynucleotide which encodes a fusion protein comprising an AML-specific antigen and the structural protein precursor (P1) of TrV or a functionally equivalent variant thereof, wherein optionally the host cell is additionally contacting with a polynucleotide comprising a sequence encoding the non-structural protein precursors (NS) of TrV or a functionally equivalent variant thereof, under conditions suitable for the entry of said polynucleotide into the cell;
   (ii) maintaining the cell under conditions suitable for the expression of the polynucleotide introduced in the cell in step (i), and
   (iii) recovering the VLPs from the culture obtained.
31. The process according to aspect 30, wherein the AML-specific antigen is inserted in the sequence encoding P1 at
   (i) a nucleotide position corresponding to an amino acid inside at least one TrV structural protein derived from P1,
   (ii) a nucleotide position corresponding to the amino terminal end of at least one TrV structural protein derived from P1, and/or
   (iii) a nucleotide position corresponding to the carboxy terminal end of at least one TrV structural protein derived from P1
   wherein said at least one TrV structural protein is the same TrV structural protein or is a different TrV structural protein, and or wherein said AML-specific antigen is the same antigen or is a different antigen.
32. The process according to aspect 31, wherein the AML-specific antigen is located between nucleotide positions which result in a fusion protein wherein the AML-specific antigen is located at a position selected from the group consisting of (numbers correspond to amino acids of protein precursor P1):
   - between amino acid positions Ala681 and Trp688 in VP1 structural protein,
   - between amino acid positions Asp841 and Thr854 in VP1 structural protein,
   - between amino acid positions Ser862 and Thr868 in VP1 structural protein,
   - between amino acid positions Leu1 and Asn14 in VP2 structural protein,
   - between amino acid positions Thr97 and Lys103 in VP2 structural protein,
   - between amino acids positions Gly246 and Val253 in VP2 structural protein,
   - between amino acids positions Pro588 to Ile595 in VP3 structural protein,
   - between amino acids positions Lys258 to Leu310 in VP4 structural protein.
33. A process for obtaining a *Triatoma virus* (TrV) VLP modified to contain an AML-specific antigen comprising the steps:
   (i) contacting a host cell with a first polynucleotide which encodes a fusion protein comprising a second member of a binding pair and the structural protein precursor (P1) of TrV or a functionally equivalent variant thereof, wherein optionally the host cell is additionally contacting with a polynucleotide comprising a sequence encoding the non-structural protein precursors (NS) of TrV or a functionally equivalent variant thereof, under conditions suitable for the entry of said polynucleotide into the cell;
   (ii) maintaining the cell under conditions suitable for the expression of the polynucleotide introduced in the cell in step (i), and
   (iii) recovering the VLPs from the culture obtained
   (iv) contacting the VLPs recovered in step (iii) with the AML-specific antigen wherein the AML-specific antigen is provided as a fusion protein with a first member of a binding pair under conditions adequate for the interaction between said first and second members of the binding pair to occur.
34. The process according to aspect 33, wherein the second member of the binding pair is inserted in the sequence encoding P1 at
   (i) a nucleotide position corresponding to an amino acid inside at least one TrV structural protein derived from P1,
   (ii) a nucleotide position corresponding to the amino terminal end of at least one TrV structural protein derived from P1, and/or
   (iii) a nucleotide position corresponding to the carboxy terminal end of at least one TrV structural protein derived from P1
   wherein said at least one TrV structural protein is the same TrV structural protein or is a different TrV structural protein, and or wherein said AML-specific antigen is the same antigen or is a different antigen.
35. The process according to aspect 34, wherein the second member of the binding pair is located between nucleotide positions which result in a fusion protein wherein the AML-specific antigen is located at a position selected from the group consisting of (numbers correspond to amino acids of protein precursor P1):
   - between amino acid positions Ala681 and Trp688 in VP1 structural protein,
   - between amino acid positions Asp841 and Thr854 in VP1 structural protein,
   - between amino acid positions Ser862 and Thr868 in VP1 structural protein,
   - between amino acid positions Leu1 and Asn14 in VP2 structural protein,
   - between amino acid positions Thr97 and Lys103 in VP2 structural protein,
   - between amino acids positions Gly246 and Val253 in VP2 structural protein,
   - between amino acids positions Pro588 to Ile595 in VP3 structural protein,
   - between amino acids positions Lys258 to Leu310 in VP4 structural protein.
36. The process according to aspect 35, wherein the first member of a binding pair is selected from the group consisting of a peptide comprising the tripeptide Arg-Gly-Asp, a peptide comprising the sequence XBBXBX, wherein X represents any amino acid and B represents Arg or Lys, and a peptide comprising the sequence XBBBXXBX, wherein X represents any amino acid and B represents Arg or Lys and a biotin acceptor peptide (BAP).
37. The process according to aspect 36, wherein the first member of the binding pair is the biotinylated BAP and the second member of the binding pair is a molecule comprising a biotin-binding region.
38. The process according to aspect 37, wherein the molecule comprising a biotin-binding region is selected from the group comprising avidin, an avidin analogue, streptavidin, and streptavidin analogue.
39. A process for obtaining a *Triatoma virus* (TrV) VLP modified to contain an AML-specific antigen comprising the steps:
   (i) contacting a host cell with a first polynucleotide which encodes the structural protein precursor (P1) of TrV or a functionally equivalent variant thereof, wherein optionally the host cell is additionally contacting with a polynucleotide comprising a sequence encoding the non-structural protein precursors (NS) of TrV or a functionally equivalent variant thereof, under conditions suitable for the entry of said polynucleotide into the cell;
   (ii) maintaining the cell under conditions suitable for the expression of the polynucleotide introduced in the cell in step (i), and
   (iii) recovering the VLPs from the culture obtained
   (iv) treating the VLPs obtained in step (iii) with a reagent that creates functional groups on the VLP thereby creating functionalized VLPs and reacting the functionalized VLPs with a funcionallized form of the AML-specific antigen which functional groups which are reactive with the functional groups present in the funcionalized VLPs or, alternatively, contacting the VLPs obtained in step (iii) with the AML-specific antigen in the presence of a bifunctional reagent that is capable of reacting with groups present in the VLP and in the AML-specific antigen thereby cross-linking the VLP and the antigen.
40. The process according to any of aspects 30 to 39, wherein the AML-specific antigen is selected from the group consisting of:
   (i) an antigen derived from the nucleophosmin 1 protein,
   (ii) an antigen derived from the WT1 protein,
   (iii) an antigen derived from the isocitrate dehydrogenase 1 protein, and
   (iv) an antigen derived from a mutant FLT3 gene comprising an internal tandem duplication.
41. The process according to any of aspects 30 to 40, wherein the AML-specific antigen comprises an epitope which is selected from the group consisting of the sequences AVEEVSLRK peptide (UniProt:E5RI98 and P06748) (SEQ ID NO: 1), the RMFPNAPYL peptide from Wilms tumor protein 1 (UniProt:P19544) (SEQ ID NO: 2), the GWVKPIIIGHHAYGDQYRAT peptide (IEDB (iedb.org) epitope ID 1445707) (SEQ ID NO: 3) or the YVDFREYEYY peptide (IEDB epitope ID 858644) (SEQ ID NO: 4).
42. The process according to any of aspects 30 to 41, wherein the AML-specific antigen comprises at least one functional group.
43. A VLP obtainable by the process according to any of aspects 30 to 42.
44. A vaccine or immunogenic composition comprising a VLP according to any of aspects 1 to 14, a polynucleotide according to any of aspects 15 to 27, or a vector according to aspect 28.
45. A VLP according to any of aspects 1 to 16, a polynucleotide according to aspects 15 to 27, or a vector according to aspect 28, for use in the treatment of an AML.
46. A VLP according to any of aspects 1 to 16, a polynucleotide according to aspects 17 to 27, or a vector according to aspect 28, for use in the treatment of the AML subtype characterized by presence of recurrent mutation of nucleophosmin-1 (NPM1).

### EXAMPLES

The following examples illustrate the invention and must not be considered as limiting the scope thereof.

To generate prototypes of VLP-based vaccines against AML-NPMc+, inventors have designed, based on structural analysis and predictions, constructs of TrV-VLPs fused to the neoantigenic motif AVEEVSLRK, corresponding to the C-terminus of NPM1 mutant A. Inventors have generally selected insertion / addition sites in flexible / exposed regions of the viral proteins, to avoid interference with the particle assembly. Such chimeric constructs thus generated are then produced in the Baculovirus / insect cells system, purified and tested in a mouse model of AML.

### Material and Methods

Several insertion sites on the TrV capsid have been selected by the inventors on the bases of structural prediction and the following criteria: a) being conformationally flexible regions of the VLP structural proteins (VP); b) being exposed on the surface of the particle; c) avoidance of icosahedral symmetry axes; d) comparison with the homologous virus Cricket paralysis virus (CrPV) in terms of C-termini length, etc. Thus, insertions at such selected sites are not expected to interfere with the assembly of the VLP particles (Figure 2).

The previously described clones for the recombinant production of TrV VLPs in the Baculovirus / insect cells system (Sanchez-Eugenia et al., J Gen Virol. 2015 Jan;96:64, International patent application published as WO2015101666A1) have been used. They correspond to the TrV ORFs codifying for the non-structural proteins (NS, ORF1) and the structural polyprotein P1 (ORF2), each cloned into the EcoRI/Notl sites of plasmid pFastBac1 from Invitrogen. P1 gives rise to the capsid viral proteins (VP) VP2, VP0 (later processed to give VP3 and the small protein VP4), and VP1. Only VP1, 2 and 3 are visible in the crystal structure of the TrV capsid (Squires G. et al. Acta Crystallogr D Biol Crystallogr. 2013 Jun;69:10262013). Their DNA sequence is described in entry Q9QEY5 (UniProtKB), but the P1 sequence differs from Q9QEY5 in the amino acid substitutions H49D in VP2 and V54M, V128I, K202E in VP3.

Fusion constructs of viral proteins with the NPM1 motif AVEEVSLRK, corresponding to the C-terminus of NPM1 mutant A (van der Lee D.I. et al., J. Clin Invest 2019, 129, p.774) are generated through PCR and conventional cloning techniques, using primers codifying for the NPM1 nonapeptide. The peptide can either be added to the original protein sequence or replace some residues of the VPs. For example, the sequence AVEEVSLRK, is added at the VP1 C-terminus, after residue Ser264, and the correctness of the clones is then checked by DNA sequencing. This particular construct is named VLP-AML1

Heterologous expression of the VLP-AML1 has been carried out at IBET Institute (Lisbon, Portugal). The clones were used to generate the *Baculovirus*, which then infect the insect cells. The cell line Sf9 was used, grown in suspension, in a 10 L bioreactor.

The VLPs were purified following the protocol described in Sanchez-Eugenia et al. (*supra*.). The procedure involved cell disruption by freeze-thaw cycles and sonication, ultracentrifugation, sucrose cushion and fractionation on a sucrose gradient. The VLPs were further purified by size-exclusion chromatography, and finally concentrated for storage at 4°C. Both the expression and the subsequent purification of the VLPs were monitored by Western Blot with an anti-VP1 antibody (Sanchez-Eugenia R. et al., J Gen Virol. 2015 Jan;96:64).

The VLP preparations (at least of wild-type) were characterized through dynamic light scattering, and negative staining transmission electron microscopy, where they displayed size and shape properties compatible with those previously described for TrV empty capsids and recombinant TrV-VLPs (Sanchez-Eugenia et al., *supra*.).

### Insertion of the epitope with the sequence AVEEVSLRK

The sequence AVEEVSLRK is inserted after Ser264 of VP1, removing the last 7 residues of the protein (Figure 2). The suitability of this truncation is based on: a) the C-terminal 7 residues are mobile / not visible in the crystal structure (Squires et al., 2013; PDB entry code 3NAP), and b) the homologous protein from CrPV is shorter in the C-terminus (Squires et al., 2013). In addition, it is considered as favourable that the motif would remain C-terminal, as in the context of the NPM1 mutant (Federici L. and Falini B. Protein Science 2013, 22, p. 545). Additionally, NPM1 structure is homopentameric (Lee et al. 2007. Proteins Struct. Funct. Bioinform. 2007, 69, 672-678), and it will display the inserted sequence following also a pentameric distribution, as is displayed in Figure 2.

To generate the construct, a polymerase chain reaction (PCR) is run, with the primers 5'- CCGGAATTC**ATG**CTCGCTGTAAATAATGTAAATATG (SEQ ID NO: 9) (Sanchez-Eugenia et al., *supra*.) and 5'-TTAAAAGCGGCCGC**CTA**TTTTCTTAAAGATACTTCTTCAACAGCAGATAGATAAAA ATTTGAGAAGGTAG (SEQ ID NO: 10). In their sequence, the restriction sites for EcoRI and Notl, respectively, are double-underlined, the START and STOP codons in bold type, and the sequence codifying for the NPM1 motif is underlined. The conditions for the PCR are: 1 µM of each primer, dNTPs (4 µM each, PCR Nucleotide Mix, Roche), 2 U of Phusion HF DNA polymerase (ThermoFisher) and approximately 150 ng of pFastBac1-P1 plasmid, in a total volume of 50 µL. The program is as follows: 1) 2' at 95 °C, 2) 30" at 95 °C, 3) 30" at 54 °C, 4) 3' at 68 °C and 5) 10' at 68 °C. Steps 2-4 are cycled 30 times.

The amplified fragment is purified with the "QIAquick PCR Purification Kit" from Qiagen and digested with EcoRI and Notl (FastDigest, ThermoFisher) during 25 minutes at 37°C. After inactivating the enzymes, the fragment is ligated, with T4 ligase (New England Biolabs) into the plasmid pFastBac1 previously digested with the same restriction endonucleases and dephosphorylated with alkaline phosphatase (Applied Biosciences). The ligation reaction is used to transform chemocompetent *E. coli* cells (DH5a strain), by heat shock. Colonies grown on LB/agar/ampicillin plates are inoculated into liquid media with 100 µg/ml ampicillin and grown until saturated. Then, the plasmid DNA is extracted from them with the QIAprep Spin Miniprep Kit of Qiagen. The presence of an insert of the expected size is confirmed through restriction analysis with EcoRI / Notl, and the plasmids corresponding to the positive clones are finally checked by DNA sequencing.

The validated plasmids are then used to generate bacmides for transfecting insect Sf9 cells, with the ratio polyprotein (P1): non-structural proteins (NS) of 1:2, and MOI (multiplicity of infection) of 0.1 pfu/cell. Cells are grown in a 10 L fermentor, reaching a viable cell concentration index (CCI) of 2 x 10⁶ cells / mL. Baculoviruses are harvested 96h post-infection through centrifugation at 3000 g for 5 minutes. The biomass pellet is then resuspended in PBS and stored at -20°C.

VLPs are purified according to Sanchez-Eugenia. et al., J Gen Virol. 2015 Jan;96:64, using the buffer 50 mM Tris / HCI pH 7.0, 10 mM NaCl, and 1 mM MgCl₂. The protocol involves breaking the cells, ultracentrifugation and filtration, sucrose cushion followed by sucrose gradient ultracentrifugations, and size exclusion chromatography with Sephacryl-500HR. The purification is monitored by Western-blot with anti-VP1 antibody. The obtained preparations are characterized by SDS-PAGE, mass spectrometry, dynamic light scattering (DLS) and negative staining electron microscopy (EM), all of them confirming that the VLPs display the expected protein composition, size, homogeneity and morphology, as previously described (Sanchez-Eugenia et al., 2015 *supra*.).

The VLPs are to be validated in ELISA immunoassays with a specific antibody against the mutated NPM1 sequence to confirm the exposure of the inserted epitope, as well as in cell-based reporter assays for characterization of innate immune receptor induced signalling cascades.

## Claims

1. A virus-like particle (VLP) comprising
(i) the viral structural proteins VP1, VP2, VP3 and VP4, or the viral structural proteins VP1, VP2, and VP0, or the structural protein precursor (P1) of *Triatoma virus* (TrV), or a functionally equivalent variant of any of the above , and
(ii) an acute myeloid leukaemia (AML) specific antigen
wherein said antigen is provided as a fusion protein with at least one of said viral structural proteins or wherein said antigen is connected to at least one of the said viral proteins by a linker region.

2. A polynucleotide encoding a fusion protein selected from the group consisting of
(i) a fusion protein comprising a structural protein from a virus of the *Dicistroviridae* family selected from the group consisting of VP1, VP2, VP3, VP4 and VP0, or a functionally equivalent variant thereof, and an AML-specific antigen, or
(ii) a fusion protein comprising a structural protein precursor (P1) from a virus of the *Dicistroviridae* family, or a functionally equivalent variant thereof, and an AML-specific antigen.

3. A polynucleotide encoding a fusion protein selected from the group consisting of:
(i) a fusion protein comprising a structural protein from a virus of the *Dicistroviridae* family selected from the group consisting of VP1, VP2, VP3, VP4 and VP0, or a functionally equivalent variant thereof, and a first member of a binding pair, or
(ii) a structural protein precursor (P1) from a virus of the *Dicistroviridae* family, or a functionally equivalent variant thereof, and a first member of a binding pair.

4. The polynucleotide according to any of claims 2 or 3, wherein the fusion protein further comprises a non-structural protein (NS) from a virus of the *Dicistroviridae* family, or a functionally equivalent variant thereof.

5. The polynucleotide according to any one of claims 2 to 4, wherein the virus of the *Dicistroviridae* family is the *Triatoma virus* (TrV).

6. A vector or a host cell comprising the polynucleotide according to any one of claims 2 to 5.

7. A process for obtaining the *Triatoma virus* (TrV) VLP modified to express an AML-specific antigen comprising the steps:
(i) contacting a host cell with a first polynucleotide which encodes a fusion protein comprising an AML-specific antigen and the structural protein precursor (P1) of TrV or a functionally equivalent variant thereof, wherein optionally the host cell is additionally contacting with a polynucleotide comprising a sequence encoding the non-structural protein precursors (NS) of TrV or a functionally equivalent variant thereof, under conditions suitable for the entry of said polynucleotide into the cell;
(ii) maintaining the cell under conditions suitable for the expression of the polynucleotide introduced in the cell in step (i), and
(iii) recovering the VLPs from the culture obtained.

8. A process for obtaining a *Triatoma virus* (TrV) VLP modified to contain an AML-specific antigen comprising the steps:
(i) contacting a host cell with a first polynucleotide which encodes a fusion protein comprising a second member of a binding pair and the structural protein precursor (P1) of TrV or a functionally equivalent variant thereof, wherein optionally the host cell is additionally contacting with a polynucleotide comprising a sequence encoding the non-structural protein precursors (NS) of TrV or a functionally equivalent variant thereof, under conditions suitable for the entry of said polynucleotide into the cell;
(ii) maintaining the cell under conditions suitable for the expression of the polynucleotide introduced in the cell in step (i), and
(iii) recovering the VLPs from the culture obtained
(iv) contacting the VLPs recovered in step (iii) with the AML-specific antigen wherein the AML-specific antigen is provided as a fusion protein with a first member of a binding pair under conditions adequate for the interaction between said first and second members of the binding pair to occur.

9. A process for obtaining a *Triatoma virus* (TrV) VLP modified to contain an AML-specific antigen comprising the steps:
(i) contacting a host cell with a first polynucleotide which encodes the structural protein precursor (P1) of TrV or a functionally equivalent variant thereof, wherein optionally the host cell is additionally contacting with a polynucleotide comprising a sequence encoding the non-structural protein precursors (NS) of TrV or a functionally equivalent variant thereof, under conditions suitable for the entry of said polynucleotide into the cell;
(ii) maintaining the cell under conditions suitable for the expression of the polynucleotide introduced in the cell in step (i), and
(iii) recovering the VLPs from the culture obtained
(iv) treating the VLPs obtained in step (iii) with a reagent that creates functional groups on the VLP thereby creating functionalized VLPs and reacting the functionalized VLPs with a funcionallized form of the AML-specific antigen which functional groups which are reactive with the functional groups present in the funcionalized VLPs or, alternatively, contacting the VLPs obtained in step (iii) with the AML-specific antigen in the presence of a bifunctional reagent that is capable of reacting with groups present in the VLP and in the AML-specific antigen thereby cross-linking the VLP and the antigen.

10. A VLP according to claim 1, a polynucleotide according to any one of claims 2 to 5, or a process according to any one of claims 7 to 9, wherein the AML-specific antigen is selected from the group consisting of:
(i) an antigen derived from the nucleophosmin 1 protein,
(ii) an antigen derived from the WT1 protein,
(iii) an antigen derived from the isocitrate dehydrogenase 1 protein, and
(iv) an antigen derived from a mutant FLT3 gene comprising an internal tandem duplication.

11. A VLP according to claim 1, a polynucleotide according to any one of claims 2 to 5, or a process according to any one of claims 7 to 9, wherein the AML-specific antigen comprises an epitope which is selected from the group consisting of the sequences AVEEVSLRK peptide (UniProt:E5RI98 and P06748) (SEQ ID NO: 1), the RMFPNAPYL peptide from Wilms tumor protein 1 (UniProt:P19544) (SEQ ID NO: 2), the GWVKPIIIGHHAYGDQYRAT peptide (IEDB (iedb.org) epitope ID 1445707) (SEQ ID NO: 3) or the YVDFREYEYY peptide (IEDB epitope ID 858644) (SEQ ID NO: 4).

12. A VLP obtainable by the process according to any one of claims 7 to 9.

13. A vaccine or immunogenic composition comprising a VLP according to any of claims 1, 10 or 11, a polypeptide according to any one of claims 2 to 5, or a vector according to claim 6.

14. A VLP according to any of claims 1, 10 or 11, a polynucleotide according to any one of claims 2 to 5, or a vector according to claim 6, for use in the treatment of an AML.

15. A VLP according to any of claims 1, 10 or 11, a polynucleotide according to any one of claims 2 to 5, or a vector according to claim 6, for use in the treatment of the AML subtype **characterized by** presence of recurrent mutation of nucleophosmin-1 (NPM1).
